# EUROPEAN PATENT SPECIFICATION

(11) **EP 3 185 757 B1**
(45) Date of publication and mention of the grant of the patent: **07.06.2023**
(21) Application number: 15788180.6
(22) Date of filing: 25.08.2015
(51) Int. Cl.: A61B 5/00, A61B 5/11, G01B 7/00, G01B 7/16

(54) **ELASTIC SENSOR**
ELASTISCHER SENSOR
CAPTEUR ÉLASTIQUE

(30) Priority: 25.08.2014 BE 201400645; 25.11.2014 EP 14194681; 14.06.2015 EP 15172004
(43) Date of publication of application: 05.07.2017
(73) Proprietor: Bainisha CVBA, 9160 Lokeren (BE)
(72) Inventor: VAN DE VYVER, Patrick, B-9160 Lokeren (BE)
(74) Representative: DenK iP bv
(86) International application number: PCT/IB2015/001742
(87) International publication number: WO 2016/030752

(56) References cited:
- WO-A1-2013/041101
- WO-A1-2014/050245
- WO-A2-2013/186693
- US-A1- 2010 036 287
- US-A1- 2011 067 253
- US-A1- 2011 241 704
- DARRYL P J COTTON ET AL: "A Multifunctional Capacitive Sensor for Stretchable Electronic Skins", IEEE SENSORS JOURNAL, IEEE SERVICE CENTER, NEW YORK, NY, US, vol. 9, no. 12, 1 December 2009 (2009-12-01), pages 2008-2009, XP011279348, ISSN: 1530-437X, DOI: 10.1109/JSEN.2009.2030709

## Description

### Field of the invention

The invention relates in general to a stretchable and flexible sensor patch for measuring and storing and/or transmitting data relating to movements (of e.g. a back or limbs of a person or an animal), and to a sensor system containing such a sensor patch and which can read and/or receive and process data. The invention also relates to a specific use of such a sensor patch. The invention also relates to an elastic object, e.g. a ball or inflatable ball, on which a sensor patch is provided or in which capacitative or resistive sensor elements are integrated.

### Background of the invention

Various portable devices are known for measuring and storing movements such as bending of knees during stepping or movements of the lower back during bending or twisting. They vary from use of a measurement strip/adhesive strip and a marker pen to the very popular technique known as EMG probing (electromyogram) wherein a number of small and light self-adhesive electrodes are applied to a body. One important disadvantage of EMG probing however is that measurement requires the use of an extra measurement system such as cameras, whereby usage is restricted to measurements in a controlled environment (e.g. in a laboratory).

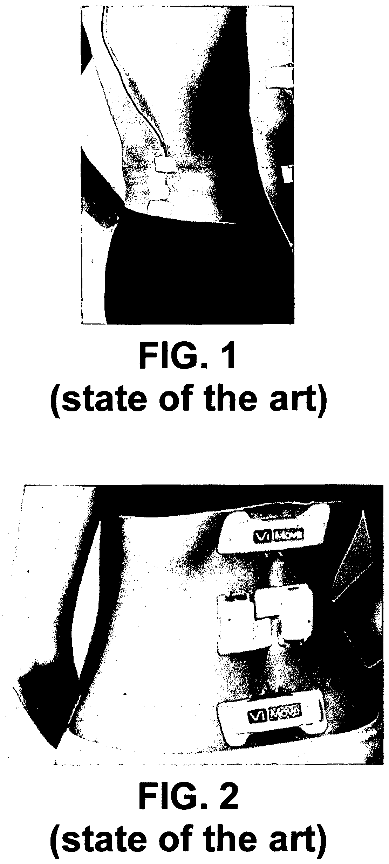

Fig. 1 shows a measurement system known as "The Bodyguard^{®} ", commercially available from the Belgian company Sels Instruments. The basic configuration consists of a strain gauge measurement sensor and a lightweight portable housing with signal processing and communication electronics. A disadvantage of this system however is that the installation can detach relatively easily, and measurement is in fact limited to a single sensor. More information on this system is available at the following link "http://www.sels-instruments.be/bodyguardsystem.html".

Fig. 2 shows a measurement system called "Vi Move8 "' by the Australian company "dorsaVi Ltd". This system contains two integrated EMG electrodes. A disadvantage of this system is that it has a housing with a non-negligible thickness, and that it consists of several separate devices which must be applied with a specific mutual interval and orientation.

Document WO 2014/050245 A1 a capacitance-type sensor sheet used for measuring an amount of stretch deformation and strain, and/or distribution of stretch deformation and strain.

DARRYL P J COTTON ET AL: "A Multifunctional Capacitive Sensor for Stretchable Electronic Skins", IEEE SENSORS JOURNAL, IEEE SERVICE CENTER, NEW YORK, NY, US, vol. 9, no. 12, 1 December 2009 (2009-12-01), pages 2008-2009, XP011279348, ISSN: 1530-437X, DOI: 10.1109/JSEN.2009.2030709, discloses a multifunctional capacitive sensor. In Cotton a single sensor is discussed. The sensor in Cotton is capable of registering strain, pressure, and finger touch. Cotton suggests that such sensors can be integrated into large area sensory skins capable of registering the shape of the object in contact with the e-skin and the pressure with which it is applied.

In view of the prior art there is a need for sensor patches which can be applied to a skin of a person or an animal and is configured for measuring data relating to movements.

### Summary of the Invention

It is an object of the present invention to create a good sensor patch which can be applied to a skin of a person or an animal and is configured for measuring and storage and/or transmission of data relating to movements.

It is also an object of the present invention to create a good sensor system for reading and/or receiving and processing the measured data.

It is also an object of the present invention to create some specific applications for such sensor patches.

These objects are achieved by a sensor patch and a sensor system and a use according to embodiments of the present invention.

In one aspect, the present invention concerns an elastic sensor patch comprising an elastic and electrically isolating film layer with a stretchability of at least 100% in all directions in the plane of the film layer (X, Y), and a plurality of elastic measurement strips attached to the elastic film layer, wherein each elastic measurement strip comprises an oblong capacitive strip with a dielectric electro-active polymer and has a stretchability of at least 50% in its length direction, wherein the sensor patch comprises a first number of at least two elastic measurement strips which are oriented mutually parallel to each other in a first direction and lie at a distance from each other in the range of 3 mm to 100 mm, and a second number of at least two elastic measurement strips which are oriented mutually parallel to each other in a second direction which intersects the first direction, wherein each elastic measurement strip comprises two electrically conductive metal film layers attached to opposite sides of the oblong capacitive strip with the dielectric electro-active polymer, wherein the two metal film layers have a geometric form which allows elastic elongation without breaking the metal film

A measurement strip may comprise at least two sensor arms which extend at least partly in two directions significantly different from each other.

The sensor arms of the measurement strip may be under a pretension.

Parts of the at least two sensor arms may mutually form an angle of at least 20°, for example an angle of at least 30°.

The elastic measurement strip may comprise a first number of at least two strips which are oriented mutually parallel to each other in a first direction and lie at a distance from each other in the range of 3 mm to 100 mm, and/or a second number of at least two strips which are oriented mutually parallel to each other in a second direction which intersects the first direction.

The sensor arms may be configured so that the sensor configuration has a cross form.

The sensor may comprise a plurality of elastic measurement strips in one sensor configuration, so that different cross-shaped measuring units can be positioned next to each other.

In one aspect, the present invention concerns an elastic sensor patch comprising an elastic and electrically isolating film layer with a stretchability of at least 100% in all directions in the plane of the film layer (X, Y) and at least one elastic oblong capacitative strip attached to the elastic film layer, wherein the strips comprise a dielectric electro-active polymer and have a stretchability of at least 50% in their length direction.

It is an advantage of dielectric electro-active polymer (DEAP) strips that they are elastic and have a capacitance corresponding to their length. By measuring the capacitance, the length (or extent of elongation) can thus be determined.

It is an advantage of a sensor patch according to the present invention that when applied to the skin of a person or animal, the stretching of the elastic strips corresponds to the stretching of the underlying skin, which in turn corresponds to movements of body parts such as e.g. movements of joints or the spinal column.

It is an advantage of DEAP strips that they can be measured up to e.g. a hundred times per second. Hence it is possible to determine the speed or acceleration of said movements with high precision.

It is an advantage of embodiments of the sensor patch according to the present invention that the elastically stretchable and flexible form allows the sensor patch to be applied (e.g. glued) onto a curved surface such as around a leg, but also around an irregular surface such as a knee.

It is an advantage of embodiments of the present invention that the sensor patch can be enclosed watertight so that the sensor patch need not be removed even in water (e.g. during showering or during swimming or if livestock are standing in the rain).

In some embodiments, the sensor patch may also comprise an integrated circuit wherein the integrated circuit is attached to the elastic film layer and electrically connected with at least one said strip (one or more) or filament or fibre based thereon, by means of elastic electric connections, wherein the integrated circuit is configured for measuring a value indicative of the elongation of said strip or filament or fibre based thereon.

It is an advantage that the integrated circuit may comprise a programmable processor. The program may be stored in non-volatile memory (e.g. embedded flash) or may be hard-coded. The integrated circuit may be an ASIC (application-specific integrated circuit).

It is an advantage of a sensor patch according to the present invention that it may have elastic electric connections e.g. in snake form or zigzag form, or interconnection of horseshoe-like form. It is furthermore an advantage of such connections that they may be applied directly onto the film layer, e.g. by printing techniques, and that they are very stretchable. Although it is possible to connect the strips in other ways, e.g. by loose wires with a length corresponding to the maximum length (in elongated state), the proposed technique of printed connections is much thinner, whereby a very thin stretchable sensor patch can be obtained.

Said at least one strip may comprise two electrically conductive metal film layers applied on opposite sides of the strips, wherein the two metal film layers have a geometric form which allows elastic elongation without breaking the metal film.

It is an advantage of such geometric forms (e.g. ripple or wave structure) that such strips allow a very great elastic stretching. In a specific embodiment, the capacitative strips are made of a material known as "PolyPower DEAP", commercially available from the company "Danfoss-PolyPower". However the present invention is not restricted to this and will also function with other structures or materials which have similar properties, and materials which do not exist at present e.g. second or later generation products of PolyPower DEAP. The metal films may have a ripple structure or a wave structure. The form of the polymer and the metal film applied thereon may be a ripple structure for example (similar to a corrugated plate structure for roof covering but much smaller). Such a structure may e.g. have a sine wave cross-section which behaves as a harmonic structure during stretching or re-contracting without breaking.

It is an advantage of a sensor patch according to the present invention that it does not consist purely of a DEAP polymer with a double ripple structure, but contains strips of such material because it allows both measurement of the elongation and also stretching of the stretchable sensor patch in two directions, while the wave structure is not equally stretchable in all directions or is practically not stretchable in one specific direction.

The at least one strip, for example the plurality of strips, may comprise a first number of at least two strips which are oriented mutually parallel to each other in a first direction and lie at a distance from each other in the range from 3 mm to 100 mm. The at least two parallel strips or filaments may e.g. lie at a relatively small distance from each other of around 3 mm or around 5 mm or around 8 mm or around 10 mm. By providing a large quantity of strips on a relatively small area, elongation can be measured with greater precision.

It is however also possible to place the strips or filaments at a relatively great distance from each other, e.g. 20 mm or 40 mm or 60 mm or 80 mm or 100 mm or even more. It is an advantage of such a configuration that the elongation can be determined over a relatively large area of the skin.

The at least one strip or at least one filament, for example the plurality of strips, may comprise a second number of at least two strips or two filaments which are oriented mutually parallel to each other in a second direction which intersects the first direction.

In one embodiment, the first and second direction stand perpendicular to each other. In a specific embodiment, the first number of strips or filaments and the second number of strips or filaments contain the same number of strips or filaments +/- 2, and the strips or filaments are applied in a herringbone structure. The strips or filaments may thus form a letter V.

It is an advantage of certain embodiments of the present invention that two DEAP strips or resistive filaments are connected together electrically, so that only 3 electrical connections suffice for measuring two DEAP strips or resistive filaments. In this way, 1/4 of the electrical connections and the number of pins of the integrated circuit can be saved.

The width (Ws) of the at least one strip or filament may be less than 5 mm.

It is an advantage of embodiments of the sensor patch that the width of the DEAP strips or filaments is less than 5 mm. Since the strips or filaments are stretchable in the length direction but not necessarily in the width direction, limiting the width of the strips or filaments has the advantage that (for an equal number of strips on a given patch area), a greater part of the patch area is not covered by the strips (in the width direction) or filaments, hence in other words the patch remains stretchable over a larger percentage of its area even in the transverse direction. This also offers the advantage that several strips or filaments can be applied to the same patch, whereby a greater number of measurement values can be obtained, whereby the movement of the underlying biostructure (e.g. joint) can be mapped more precisely.

The sensor patch may furthermore comprise an adhesion layer for sticking the film layer to a skin of a person or animal. It is an advantage of embodiments of the present invention that the adhesion layer is already present in the patch so no separate adhesive need be applied. The sensor patch may furthermore comprise a memory for storage of measured values. The memory may for example contain the measured capacitance values (representative of the length of the strips) and/or values connected therewith, such as the first derivative of these measurement values (representative of the speed of elongation or shrinkage), or the second derivative (representative of acceleration), or the third derivative (representative of shocks, jerks, twitches etc.).

The memory may be an internal memory of the integrated circuit. It is an advantage of embodiments of the sensor patch that the memory is an internal memory, because in this way the integration may be increased i.e. a smaller surface area is required with fewer interconnections, a lower cost price, a reduced chance of detachment of components etc. However the memory may also be an external memory, e.g. a memory with a serial interface (e.g. single-wire connection or I2C bus etc.). The memory may be a volatile memory (e.g. RAM) or a non-volatile memory (e.g. FLASH). Since an energy source is present, in principle a volatile memory is sufficient.

The memory may have a data storage capacity which is sufficiently large for storing data from a measurement over at least 8 hours. Depending on application, a smaller or a larger storage capacity may be required. During measurement, typically 10 to 100 samples are taken per second and stored in the memory, but fewer than 10 samples per second is also possible. The higher the sampling rate, the more precise the determination of the movement, speed, acceleration etc.

The sensor patch may have an energy source which is connected with the integrated circuit in order to create an electrical feed to the integrated circuit. The energy capacity of the energy source and the storage capacity of the memory may be selected as a function of application. They largely determine the autonomy of the sensor patch. If the sensor patch for example is used constantly in the vicinity of a receiver, a patch with a small memory (e.g. 8 kB RAM or even less) may suffice. If the sensor patch however is used autonomously for a longer period (e.g. several hours), (for example when attached to the leg of a cow which will graze in the field for several hours), then preferably a greater energy storage capacity and a greater memory capacity are used.

The energy source may be rechargeable and the sensor patch may furthermore comprise a charging circuit for wireless charging of the rechargeable energy source. It is an advantage of embodiments of the sensor patch according to the present invention that a rechargeable or chargeable energy source is used, because such sensor patches may have a longer autonomy, be thinner, be elastic over a larger part of their surface area, and have a lower mass.

The recharging circuit may be provided to absorb electromagnetic energy and store this in the energy source. The recharging circuit may e.g. be provided with a sub-circuit for RF coupling to an external energy source, or with a sub-circuit for capacitative coupling to an external energy source.

It is an advantage of embodiments of the present invention that charging can take place contactlessly because this allows watertight enclosure of the sensor patch.

The energy source may comprise a plurality of electrical energy cells connected together by means of an elastic electrical connection. The energy cells or energy storage elements may e.g. be small capacitors formed by two metal electrodes (e.g. Al and/or Cu) and a dielectric in-between. These capacitors may e.g. be applied between two stretchable and flexible silicone layers. It is an advantage of the use of an elastic energy storage structure over e.g. button cell batteries that a larger surface area of the sensor patch is elastic, and usually the sensor patch may be thinner and the capacitors are (re)chargeable, while an alkaline button cell battery must be connected e.g. during production of the sensor patch. In the latter case, it is also possible to restrict the energy consumption by suitable software, e.g. by starting measurement only after activation via a suitable RF command.

The sensor patch may comprise an elastic stretchable and flexible protective layer to protect the at least one strip and optionally also the integrated circuit where present. The protective layer may be applied on the film layer so that the film layer is protected against the environment. It is an advantage of the present invention that the protective layer consists of an elastic and electrically isolating material, such as e.g. silicone rubber, because this not only allows elastic stretching of the sensor patch but also protection of the components and also allows wireless transmission through the protective layer for both energy (in the case of a rechargeable energy source) and data.

The protective layer may be a watertight protective layer. It is advantageous to provide the patch with a watertight protective layer, or rather for the entire sensor patch to be watertight (between the elastic film layer and the protective layer), because this allows the patch to remain attached to the skin of a person for several days even during sweating or washing or swimming. The patch can even provide measurements during swimming. Such a watertight sensor patch is also advantageously applied to the skin of an animal, so that the sensor patch is not damaged even if the animal becomes wet or lies in a damp place. Keeping the patch fitted at the same site has the advantage over repeatedly removing and reapplying the patch that the location of the patch remains precisely the same, whereby consistency of data (e.g. before and after washing) remains guaranteed.

The sensor patch may have a mass (m) of less than 100 g, preferably less than 50 g. It is an advantage of such a patch that it is not or scarcely perceptible and that e.g. it allows unnoticed and unhindered movement, without the person or animal to which the patch is attached behaving differently because of the presence of the sensor patch.

The force required to stretch the sensor patch by 50% in any direction in the plane of the sensor patch (X, Y) may be less than 2.0 Newton. It is an advantage of such a patch that it allows unnoticed and unhindered movement, without the person or animal to which the patch is applied restricting movements or having to apply more force than normal because of the presence of the sensor patch.

The sensor patch may have a thickness (d) which is less than 10 mm, for example less than 1 mm, over its entire surface area. Preferably the thickness everywhere is less than 5 mm, more preferably less than 3 mm, most preferably less than 2 mm. It is an advantage of a stretchable patch with such a low thickness that it presents little or no hindrance, and/or is not perceptible or only slightly perceptible. The reader will immediately note that a stretchable and flexible patch of e.g. 5 mm thickness on the back causes far less hindrance than a patch with a non-deformable plastic read module of e.g. 1 cm thickness. In the first case, the user may e.g. rest against a backrest unhindered, in the second case he cannot. Also the chance of shifting and/or losing the patch is considerably reduced. The sensor patch may furthermore comprise a wireless transmitter circuit functionally connected to the integrated circuit for wireless transmission of data, wherein the integrated circuit is configured for wireless transmission of data from the memory. It is an advantage of a sensor patch with a wireless or contactless transmission circuit that the measurements can be read even during use of the sensor patch without disrupting the movement.

It is an advantage of an RF transmitter circuit that the RF signal can be transmitted over a relatively large distance (e.g. of the order of 10 to 50 metres). It is an advantage of an RF transmission circuit over a physical connector that no physical contact is required between the sensor patch and the read unit in order to exchange the data, whereby the chance of damage is drastically reduced. It is an advantage of RF signals that they can be transmitted through the protective layer, whereby a watertight seal of the sensor patch is possible. The transmission circuit may be configured for transmitting an RF signal. It is an advantage to use an RF signal because this allows data to be transmitted over a relatively large distance (e.g. 10 to 50 meters) from the receiver. It is an advantage to use a standardised protocol, such as e.g. Zigbee or Bluetooth, because this technology is mature and available in various integrated circuits as a single chip. However it is also possible to use another RF signal e.g. an analogue-modulated signal (e.g. AM modulation, FM modulation etc.). The frequency band used may be any permitted frequency band, but one of the ISM bands is preferred.

A secondary advantage of RF communication is that most digital communication protocols such as Zigbee and Bluetooth allow two-way communication, whereby e.g. it is also possible to carry out software upgrades or e.g. transmit commands or settings for optimising a specific sensor patch for a particular purpose. Thus e.g. the sampling frequency can be adjusted via the RF interface, or a start command or stop command can be given for starting or stopping the sampling. In this way energy can be saved.

The transmission circuit may be configured for transmitting a signal by capacitative coupling. Capacitative coupling is another way of transmitting data wirelessly, however this is considerably less practical than RF communication. Nonetheless, data transmission is possible via capacitative coupling, e.g. by keeping a read unit close by the patch, e.g. less than 1 cm away. Capacitative coupling through the isolating protective layer is possible and also has the advantage that the risk of damage to the internal circuit (e.g. due to EMC) or the risk of oxidation of the contacts is minimised, and that watertight protection of the sensor patch is possible.

The sensor patch may furthermore comprise a temperature sensor and the integrated circuit may be provided with an algorithm for storing the measured temperature in the memory, and/or for compensating the measurement values taking into account the temperature measured. It is an advantage for the sensor patch to have a temperature sensor and a temperature compensation algorithm because this allows an improvement in the precision of the measurement values.

The sensor patch may be constructed such that the measurement strip on the sensor patch is configured such that the sensor arms are directly connected together via elastic conductors and where applicable a centrally integrated circuit. The sensor arms are thus connected together but not necessarily via sensor material. The present invention also concerns the use of the sensor patch as described above, wherein the sensor patch is attached to the skin of an animal to measure prenatal contractions. It is an advantage of embodiments of the present invention that the sensor patch is ideal for measuring prenatal contractions of e.g. a cow or a horse or other livestock. In this case the use of RF signals is a huge advantage because this allows precise monitoring of the animal remotely, which causes less stress to the animal. By monitoring the animal in good time before the birth, optimum treatment can be provided and better intervention is possible if problems threatens to arise. The sensor may be connected to a monitoring system to allow early identification of an imminent birth.

The present invention also concerns the use of the sensor patch as described above, wherein the sensor patch is applied to the skin of an animal, namely at one of the joints on one of the legs of the animal, for measuring step movements of an animal. It is an advantage of embodiments of the present invention that the sensor patch is ideal for measuring step movements (the gait) of an animal, namely a cow or a horse or other livestock. Here too the use of RF signals is a huge advantage because this allows precise monitoring of the animal remotely without hindering the movement of the animal. To increase the distance at which readout is possible, so-called "repeaters" may be placed for example around the periphery of a field. By monitoring the step movements primarily over a longer period (e.g. several weeks or even months), in particular lameness or injury, but also other diseases and/or problems can be detected early, allowing early intervention in order to prevent more serious disorders.

The use may be to detect lameness in livestock. The sensor system according to the present invention is ideal for observing (and analysing the development) of step movements of livestock for early detection of lameness or injury. Livestock means e.g. cows or horses.

The present invention also concerns the use of the sensor patch as described above wherein the sensor patch is applied to the skin of a person, namely on the back at the spine, for measuring movements of the back. It is an advantage of embodiments of the present invention that the sensor patch is ideal for measuring movements of a backbone (spinal column) of a person or animal such as e.g. a cow or a horse. Preferably the stretchable sensor patch is here applied over at least part of the skin above the spinal column. Depending on situation, above all the lower back or the upper back (e.g. between the shoulder blades) or both may be monitored. Typical dimensions for such a stretchable sensor patch are 50 cm x 10 cm, or 75 cm x 10 cm, or 100 cm x 10 cm, but other dimensions may also be used. The use of two sets of parallel strips (e.g. in the form of a herringbone) is particularly advantageous here because this allows very precise mapping of all possible movements of the back. The orientation of the strips in this case is preferably selected such that the herringbone (V-shape) is directed towards the starting leg, and the two "legs" of the V stand almost symmetrically on opposite sides of the backbone. Alternatively however, a sensor may be used with one or more sensor strips in one direction, in order to measure or monitor single movements.

The sensor patch may be applied to said skin in pre-stretched form. This offers the advantage that the strips can measure both positive and negative values (i.e. stretching of the skin and shrinkage). Since the force necessary to stretch the sensor patch is minimal (see above), it can easily be applied in slightly stretched state and the chance that the sensor patch will detach is minimal. To apply the patch with a suitable pretension, for example a suitable printing (e.g. an ellipse form) may be applied to the sensor patch which must be pressed onto the skin when the sensor patch, on stretching, shows a circle shape.

The present invention also concerns a sensor system comprising the sensor patch as described above, a processing system with a receiver configured to receive data transmitted by the sensor patch and with a calculation unit for processing the received data, and with a read unit for displaying the processed data. The processing system may e.g. be a laptop or PDA or tablet or Smartphone or similar with a wireless interface, e.g. with Bluetooth functionality, and provided with the necessary software for reading the data from the stretchable sensor patch. This laptop or PDA or tablet or Smartphone or similar differs from known laptops in that it contains a specific software program for further processing and interpretation of the data, e.g. by deriving from the measurement data derived parameters such as speed and acceleration or force and shock. The processing system may also be configured to combine the data from several sensor patches and analyse this using a mathematical model and/or a biomechanical database, so that e.g. the combined vertical and horizontal movement of a hoof of a foreleg of a cow can be determined and converted into a graph. The result of this analysis may be shown on the display or monitor or screen. In one aspect, the present invention also concerns a sensor system which comprises a sensor patch for monitoring the movement of the back, wherein the sensor patch is a sensor patch as described in one of the embodiments above, and wherein the sensor system also comprises a processor which is coupled directly or wirelessly to the sensor and is able to identify, on the basis of the movement pattern of the back, any pain moments and/or pain positions. The sensor system may also be coupled to a spinal electro-stimulation system and can send control signals to such a system for performing controlled spinal electro-stimulation. The present invention also concerns a corresponding method.

In another aspect, the present invention concerns a sensor patch based on an elastic and electrically isolating film layer, a temperature sensor attached to the film layer and a sticky gel.

In another aspect, the present disclosure concerns a sensor system in the form of a sock, collar or sleeve to be pushed over a limb of a person or animal, wherein the sock, collar or sleeve is typically made of an elastic material such as for example textile or a textile-like material or an elastomer material, the sensor system further comprising a sensor embedded in or attached to the elastic material, and wherein the sensor comprises at least one elastic oblong capacitive strip, wherein the capacitative strip comprises a dielectric electro-active polymer.

In yet another aspect, the present disclosure concerns a garment with an integrated sensor, wherein the integrated sensor is at least one elastic oblong capacitive strip, wherein the capacitative strip comprises a dielectric electro-active polymer. This capacitative strip may have a stretchability of at least 50% in the length direction.

In yet another aspect, the present invention concerns an elastic object for performing physical exercises for body training or rehabilitation, wherein the elastic object is compressed during the physical exercises, wherein the elastic object comprises a sensor which contains at least one elastic oblong capacitive strip, wherein the capacitative strip is a dielectric electro-active polymer, and/or which comprises at least one elastic oblong resistive filament e.g. a monofilament.

The sensor may be attached to the elastic object as a patch. Alternatively, not according he invention, the sensor may be integrated in the elastic object, for example in the elastic material from which the object is made. Further properties may be for example such as those of the sensor patch described above. The elastic object may be a ball.

In a further aspect, the present invention concerns an elastic object, such as for example a ball or an inflatable object, on which a sensor is positioned or in which a sensor is integrated which comprises at least one elastic oblong capacitive strip, wherein the capacitative strip is a dielectric electro-active polymer, and/or which comprises at least one elastic oblong resistive filament e.g. a monofilament.

The sensor may be attached to the elastic object as a patch. Alternatively, not according he invention, the sensor may be integrated in the elastic object, for example in the elastic material from which the object is made.

In some embodiments, a matrix of capacitative strips of dielectric electro-active polymers and/or elastic oblong resistive filaments, e.g. monofilaments, may be provided over a part or the whole of the elastic object, so that a pressure or distribution of pressure over the elastic object, and where applicable other parameters, can be determined.

It is an advantage of embodiments of the present invention that these can be used for e.g. determining pressure or pressure distribution in a large number of applications, such as for example pressure determination in a balloon on the performance of physical exercises or as part of kinesitherapy, pressure determination in a stent balloon, or pressure determination in any application.

Specific and preferred aspects of the disclosure are disclosed in the attached independent and dependent claims, which define the invention.

Features of the dependent claims may be combined with features of the independent claims and with features of other dependent claims as indicated and not merely as expressly stated in the claims.

To summarise the invention and the achieved advantages relative to the prior art, certain objectives and advantages of the invention are described above. It should however be understood that not necessarily all these objectives or advantages can be achieved by each specific embodiment of the invention. Hence for example, persons skilled in the art will recognise that the invention may be incorporated or implemented in a manner which achieves one advantage or a group of advantages as described herein, without necessarily achieving other objectives or advantages which may be achieved or suggested herein. These and other aspects of the invention will become clear from and clarified by reference to the embodiment(s) described below.

### Brief Description of the drawings

The invention will now be explained in more detail as an example, with reference to the attached figures in which:
Fig. 1 shows a measurement system known in the prior art.
Fig. 2 shows another measurement system known in the prior art.
Fig. 3 shows an embodiment of a sensor patch, wherein part of the elastic protective layer (shown in black) has been removed for illustrative purposes.
Fig. 4 shows a sensor patch according to the present invention.
Fig. 5 shows an example of a man with an embodiment of a sensor patch according to the present invention applied to his backbone.
Fig. 6 shows stretchable electrical connections attached to an elastic substrate as known in the prior art.
Fig. 7 shows an example of a flexible substrate with electronic components as known in the prior art.
Fig. 8 shows an example of a flexible and stretchable battery as known in the prior art.
Fig. 9 shows the basic elements of a conventional electrical capacitor as known in the prior art.
Fig. 10 (left) shows an example of an elastic structure with a dielectric electro-active polymer (DEAP) which is located between two electrodes with a wave structure, as known in the prior art. Fig. 10 (right) shows a function of the electrical capacitance of this structure as a function of length (when elongated).
Fig. 11 shows an example of measurement data (displacement) and derived measurement data (speed) which can be obtained with embodiments of the sensor patch according to the present invention.
Fig. 12 shows an example of a graph depicting the angular speed for the forelegs of an animal on the basis of an average of angular speed measurements and the effective angular speed for this movement.
Fig. 13 shows an example of a graph of a normal percentage cycle of measurements of a knee flexion angle measured during one step.
Fig. 14 shows several measurements such as those in Fig. 13 measured on different steps.
Fig. 15 shows a cow, wherein several useful locations on the skin are indicated at which a sensor patch according to the present invention may be applied in order for example to perform a step analysis.
Fig. 16 is a diagrammatic depiction of the forelegs (left) and hind legs (right) of the cow in

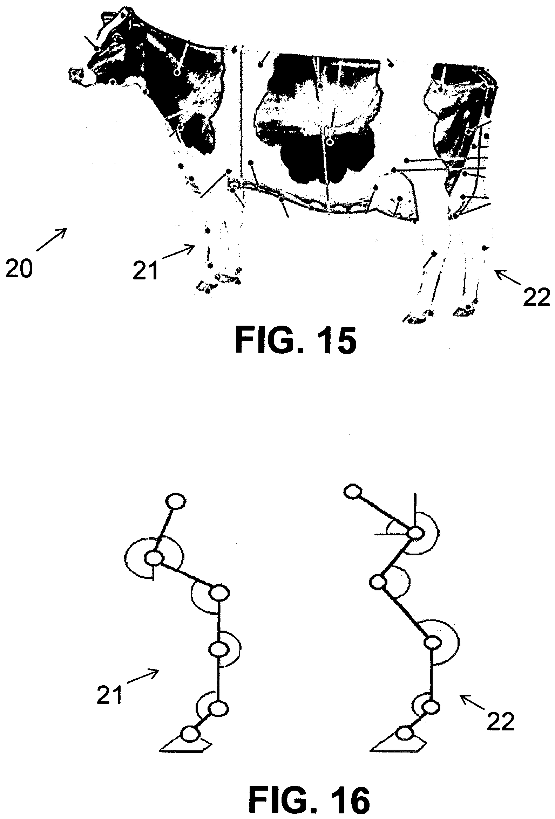

Fig. 15, wherein the joints of each leg are shown.

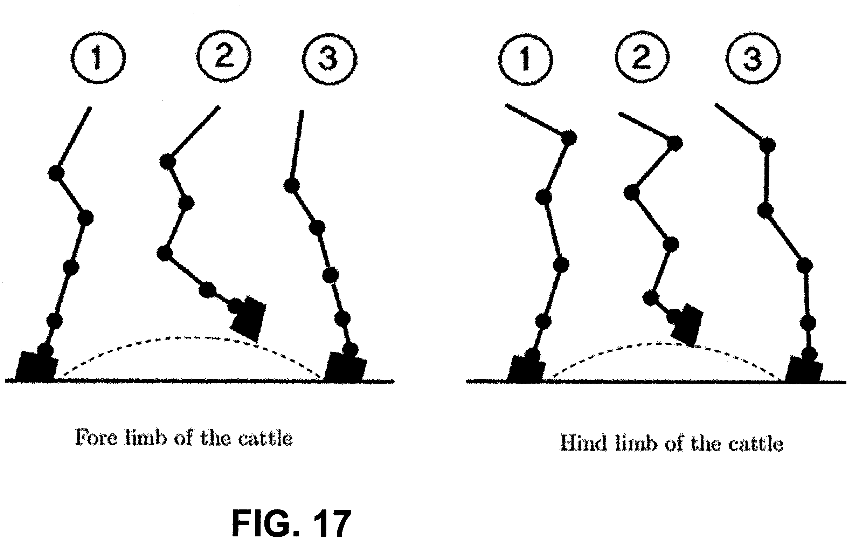

Fig. 17 shows some joints of the foreleg or hind leg of the cow during stepping, for which measurements can be performed.

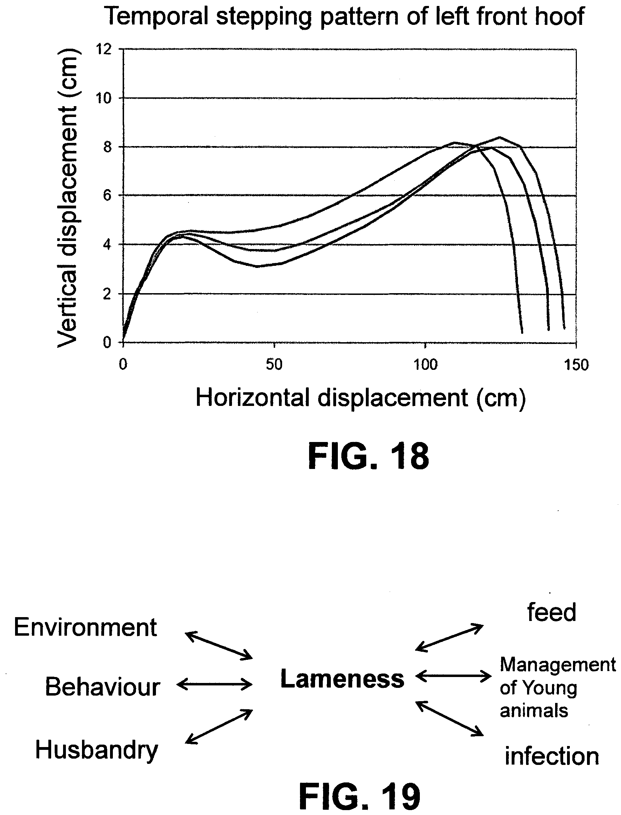

Fig. 18 is a depiction of the degradation which occurs in the stepping pattern and which is indicative of a particular clinical picture.
Fig. 19 shows a connection between lameness (also injury) and various other aspects which are important in livestock farming.

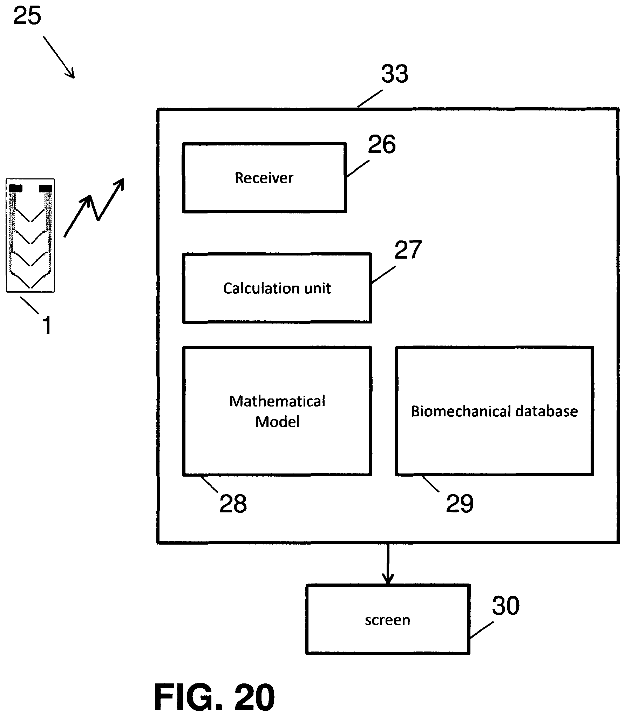

Fig. 20 shows a block diagram of a sensor system for measuring movement data, and the processing and display thereof.

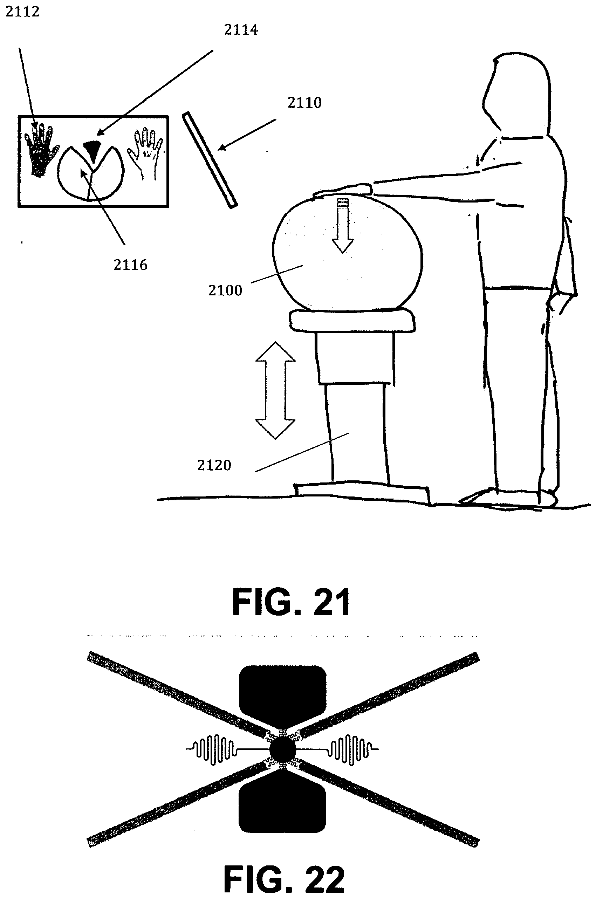

Fig. 21 shows an example of an elastic ball with sensor

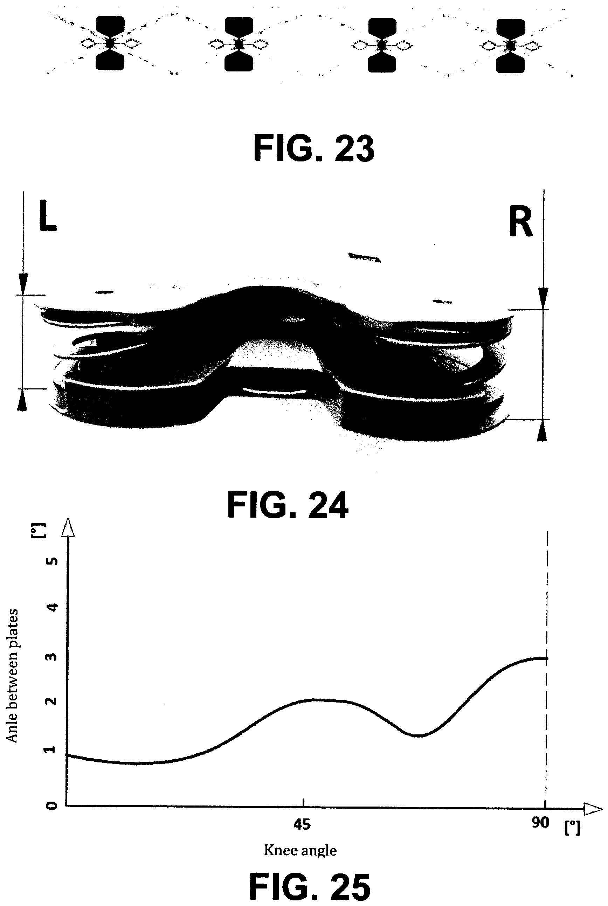

Fig. 22 and Fig. 23 show two specific configurations for back sensors
Fig. 24 illustrates a balancer and Fig. 25 illustrates a measurement of an angle between balancer components as a function of the angle of the knee, as obtained with a set of components according to an embodiment of the present invention.

The figures are merely diagrammatic and not limitative. In the figures, the dimensions of some components may be exaggerated and not shown to scale for illustrative purposes. The dimensions and relative dimensions may sometimes not correspond to the actual practical embodiment of the invention. Reference numbers in the claims may not be interpreted as restrictive of the scope of protection.

In the various figures, the same reference numbers refer to the same or equivalent elements.

### Detailed Description of Embodiments of the Invention

The present invention will be described in relation to particular embodiments and with reference to specific drawings, however the invention is not limited thereto but is restricted merely by the claims.

It should be noted that the term "comprises" as used in the claims should not be interpreted as restricted to the means described thereafter; this term does not exclude other elements or steps. It should be interpreted as specifying the presence of the features, values, steps or components to which it refers, but does not exclude the presence or addition of one or more other features, values, steps or components, or groups thereof. Hence the scope of the expression "a device comprising means A and B" should not be restricted to devices which consist only of components A and B. It means that in relation to the present invention, A and B are the only relevant components of the device.

Reference throughout this specification to "one embodiment" or "an embodiment" means that a specific feature, structure or characteristic described in connection with the embodiment is included in at least one embodiment of the present disclosure.

Hence the occurrence of the expressions "in one embodiment" or "in an embodiment" at various points throughout this specification need not necessarily always refer to the same embodiment although it may do so. The specific features, structures or characteristics may also be combined in any suitable manner, as will be clear to the average expert on the basis of this disclosure, in one or more embodiments.

Similarly, it should be appreciated that in the description of exemplary embodiments, various features are sometimes grouped together in a single embodiment, figure or description thereof with the aim of streamlining the disclosure and assisting the comprehension of one or more of the various inventive aspects. This method of disclosure should also not be interpreted as a reflection of an intention that the invention requires more features than explicitly stated in each claim. Rather, as the following claims will show, inventive aspects are present in less than all features of one single embodiment previously disclosed. Thus the claims following the detailed description are hereby explicitly included in this detailed description, with each independent claim as a separate embodiment of this invention.

Also, while some embodiments described here include some but not other features included in other embodiments, combinations of features of different embodiments form different embodiments, as will be understood by the person skilled in the art. For example, in the following claims, any of the embodiments described may be used in any combination.

In the description provided here, numerous specific details are highlighted. However it should be understood that embodiments may be implemented without these specific details. In other cases, well-known methods, structures and techniques are not described in detail, in order to keep this description concise.

In this document, the words "lameness" and "injury" are used synonymously.

Where the present invention refers to "elongation" or "stretching" or "displacement" or "movement", reference is made to a movement of a person or animal, e.g. a movement of a joint or the spinal column, and/or the associated stretching of the skin, and/or the associated stretching of the sensor patch applied to the skin. This "stretching" of the sensor patch may therefore reflect various "underlying" movements, e.g. extension or stretching of a knee or elbow or other joint, bending or stooping or stretching of the back, depending on the place and orientation where the sensor patch is applied.

Figure 1 shows a measurement system known as "The Body Guard^{®}", commercially available from the Belgian company "Sels Instruments". This system allows measurement of an elongation of a strain gauge which is glued to the skin. A disadvantage of this system is that it is not intended for measuring expansion or stretching or displacement with high density (or resolution) since only one strain gauge is provided. Furthermore, the system is not particularly useful for measuring elongation in several directions.

Figure 2 shows a measuring system known as "Vi Move^{®}", commercially available from the Australian company "dorsaVi Ltd". Insofar as known to the inventors of the present invention, in this system tilting is measured of the individual components relative to the horizontal and relative to each other, and the underlying elongation or displacement or curvature is not measured directly but derived/calculated from the tilt data. The accuracy of the measurements depends greatly on accurate positioning. In addition, the speed with which measurements can be carried out is limited. Apart from the precise application of the individual components, perhaps the greatest disadvantage of this system is that the housing on the patch is quite thick (of the order of 10 mm) and inflexible, whereby the system can be a hindrance during certain activities (such as e.g. sitting against the backrest of a chair).

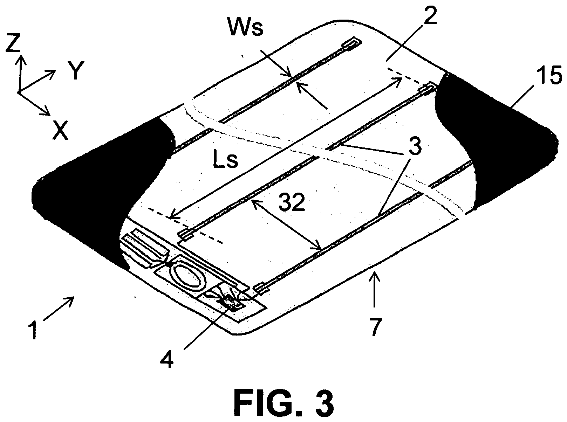

Figure 3 shows an example of a stretchable and flexible sensor patch 1 .

The sensor patch 1 comprises an elastic and electrically isolating film layer 2 with a stretchability of at least 100% in all directions in the plane of the film layer X, Y, preferably at least 200% or even at least 300%. A suitable material for this film layer 2 may for example be Tegaderm by 3M, or EU50 by Smith & Nephew, although the film layer is not restricted to these materials. The thickness of the film layer depends on the application. For a back sensor, a thin film layer will be used, whereas for an animal application a thick patch is used on a basis of sticky gel.

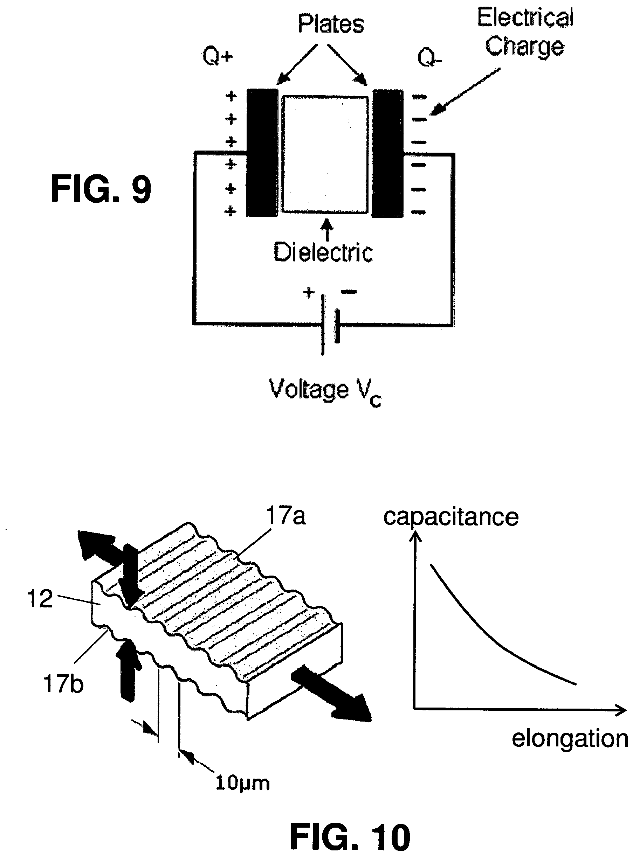

At least one, for example one or a plurality of elastic oblong strips 3 is attached to this elastic film layer 2. This at least one strip 3 to the film layer 2 may be fixed in many ways, e.g. by stitching, gluing, clamping etc. The strips 3 may e.g. be attached to the underlying film layer 2 over their entire length or only at the ends, or at several sites including the ends. The particular glue used is typically selected specifically as a function of the application. For human skin contact, specific glues may be used which are already available and which differ for example from the glue used for animal applications, since animal skin is not as smooth. According to one important aspect, the strips 3 are electric capacitors, wherein the capacitance is a measure of the length of the elongated strip. Therefore the strips 3 comprise an electrically conductive top layer and an electrically conductive bottom layer, with in-between a layer of dielectric electro-active polymer, referred to below as "DEAP" for short. Furthermore, it is important that the strips 3 have an elastic stretchability of at least 50% in their length direction. Figure 10 shows one possible specific embodiment, but the invention is not limited thereto and other strips with similar properties may also be used.

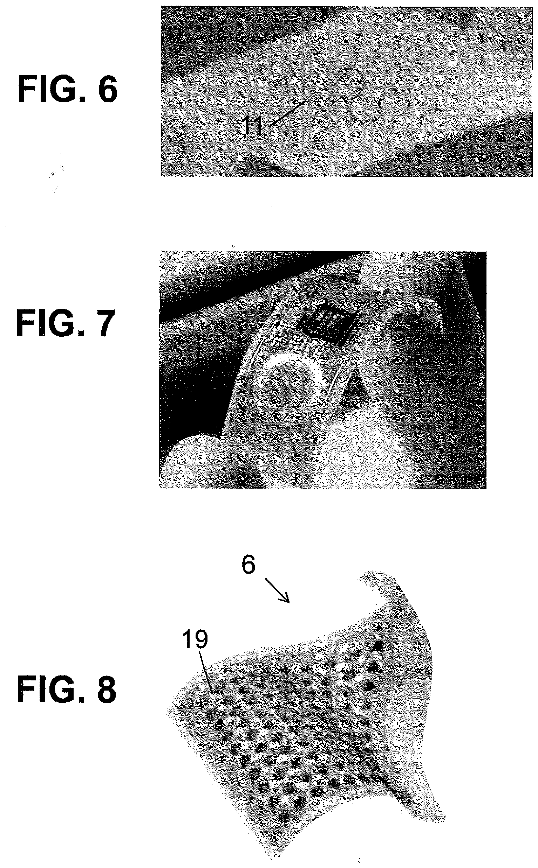

According to some embodiments, instead of strips also at least one elastic oblong resistive monofilament may be used. The elastic oblong resistive filament may be a thermoplastic elastomer with a resistivity which changes as a function of the length of the filament. In some embodiments, the resistive filament comprises 50% by weight carbon powder. The one or more resistive monofilaments may form one or more fibres. Furthermore, an integrated circuit 4 may be attached to the elastic and electrically isolating film layer 2 of the sensor patch 1 of figure 3, which circuit is electrically connected to the plurality of said strips 3 by means of stretchable electrical connections 11 (see figure 6 for an example of such connections). The integrated circuit 4 is typically provided with the necessary interfaces for measuring the capacitance of the strips 3, one or more analogue-digital converters (ADCs) for digitising the measured value, and a programmable processor which is provided with an algorithm for storing the measured capacitance value in a memory, either as such or by first converting this into a length measurement (e.g. using a graph as shown in figure 10 right) and then storing the length measurement in the memory.

Embodiments of the sensor patch 1 according to the present invention may have different memories both with regard to type (RAM, FLASH) and with regard to storage capacity.

Where applicable, the integrated circuit may be provided with an algorithm (in hardware or software or combination of the two) for using data compression to save memory. The compression may be loss-free (e.g. entropy coding, adaptive coding such as DPCM or ADPCM etc.) or may be loss-making (e.g. quantisation). Compression may also take place in time, e.g. by linear compression of a number of samples, or by run length compression, or by subsampling, but other suitable compression techniques may also be used.

Optionally, the integrated circuit 4 may furthermore comprise a temperature sensor and the processor may furthermore be configured to store the measured temperature value also in the memory and/or to convert the measured capacitance values into length measurements, taking into account the measured temperature (temperature compensation). Preferably, the memory is also integrated in the integrated circuit, but a separate temperature sensor is also possible e.g. a diode. Preferably, the integrated circuit is provided with a program which regularly measures the temperature (e.g. with a frequency in the range of 2x per second to 1x per minute, preferably in the range of 1x per second to 1x per 10 seconds, preferably around every second). The measured temperature may also be stored in the memory and if required also transmitted with the measurement data, so that the receiver can perform the temperature correction. Alternatively, the temperature is not also stored in the memory and not transmitted with the data, but the temperature correction is carried out in the sensor patch itself.

Optionally, the integrated circuit 4 may furthermore be provided with or connected to a clock, e.g. a real-time clock which can retain a date and time. If present, the date and time can also be stored in the memory e.g. as time-stamps for each measurement, or per block of a predefined number of measurement values (e.g. one time-stamp per group of measurement values corresponding to one second).

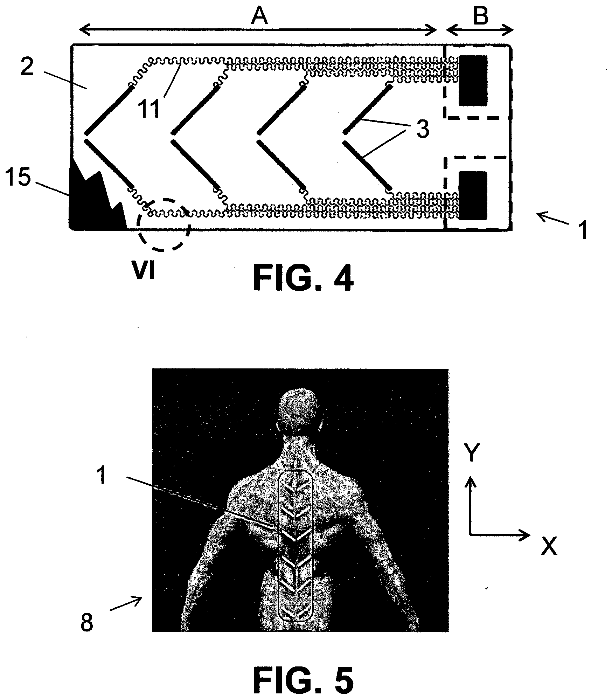

In some embodiments, the integrated circuit 4 is not itself stretchable or flexible, and for example is placed at the edge or corner of the elastic film layer 2, as shown in the sensor patch 1 of figure 3 and the sensor patch 1 of figure 4. The presence of one or more non-stretchable or inflexible components however need not be a hindrance for the bending or stretching of the rest of the film surface 2 where the elastic and capacitative DEAP strips 3 or the elastic monofilaments are situated. Alternatively, where available use may be made of a stretchable and/or flexible integrated circuit.

The sensor patch 1 according to the present invention may furthermore comprise an energy source for obtaining the electrical supply (e.g. an electrical voltage) to the electronic components (including the integrated circuit). The energy source may e.g. be of a non-elastic type such as e.g. a button cell battery of a known type (e.g. alkaline or lithium or rechargeable). In this case, the battery is preferably situated at the edge or a corner or an end of the sensor patch 1 (such as e.g. the black zones in figure 4). However, specific embodiments of the sensor patch 1 according to the present invention may also comprise a "flexible and stretchable battery" such as e.g. shown in figure 8, but the invention is not restricted thereto. The energy source 6 may comprise a plurality of individual electric energy cells 19 connected together by means of stretchable and flexible electrical connections (not shown in figure 8). The energy cells 19 may be electrical capacitors.

The energy source may also be another energy source, such as for example an ambient backscatter system which converts ambient radiation into energy, or a system which uses an electrochemical process to convert perspiration into energy.

In the case where the energy source is rechargeable, the sensor patch 1 preferably also comprises a charging circuit or recharging circuit (not shown) which is provided for wireless charging of the chargeable energy source. A suitable form of wireless charging is the use of capacitative coupling to an external charging circuit. Another suitable form of wireless charging is by means of RF radiation. Circuits for charging by capacitative coupling are known amongst others from electric toothbrushes and need not be explained further. Circuits for charging via RF radiation are known amongst others from card readers (so-called "badge readers") and also need not be explained further.

The sensor patch 1 according to the present invention may comprise further means for wireless transmission of the measured data by means of wireless communication, e.g. using a protocol such as Bluetooth or Zigbee, both of which work in the ISM band, but the invention is not limited to these, and other protocols may also be used, even a specific protocol.

The sensor patch 1 according to the present invention may furthermore comprise an adhesion layer 7 for attachment of the sensor patch 1, more specifically the film layer 2, to the skin of a person or animal. This adhesion layer is preferably a self-adhesive layer of medical quality. In some cases, an adhesion layer may be used which is specifically configured for application to animal skin, where an adequate degree of adhesion is often difficult to obtain. The adhesion layer may be applied over the entire surface of the film layer or may merely be applied at the periphery of the sensor patch, e.g. in the form of a double-sided adhesive strip. In the case where the sensor patch 1 is intended to be applied to a leg of an animal, e.g. an extra strong adhesion layer may be used in order to reduce the chance of full or partial detachment of the sensor patch 1. Although it is advantageous if the adhesion layer is already present, in some embodiments this may also be applied separately. The sensor patch 1 according to the present invention may furthermore comprise an elastic, stretchable and flexible protection layer 15 for protection of said film layer 2 and the above-mentioned components (such as the DEAP strips 3 or other capacitative strips or resistive monofilaments and the integrated circuit 4). In figure 3, the adhesion layer 7 is underneath (not visible) and the protective layer 15 is on top (partly removed for illustrative reasons). In figure 4, the adhesion layer is at the back (not visible) and the protective layer 15 has been largely removed (except for the bottom left-hand corner) for illustrative reasons. This protective layer 15 may be applied as a coating. In certain embodiments, the protective layer is a watertight protective layer. The protective layer may where applicable comprises several layers.

In other words, said components (the integrated circuit 4, the elastic DEAP strips 3 or other capacitative strips or resistive monofilaments, the elastic electrical connections, the battery (flexible or not), the charging circuit (where present), the RF transmission module etc.) may all be situated between the elastic film layer 2 and the elastic protective layer 15, and apart from any non-flexible electronic components (such as e.g. the integrated circuit 4) situated at an edge or end or at one or more corners of the sensor patch 1, the majority (e.g. more than 80%) of the surface area of the plastic sensor 1 is elastic, stretchable and flexible. Alternatively, the other components such as electronic components are also selected flexible.

The sensor patch 1 may furthermore, according to the present invention, also comprise EMG electrodes with a read circuit as known in the prior art. This read circuit may form part of the above-mentioned integrated circuit or may be a separate read circuit.

The film layer 2 and any protective layer may determine the form of the sensor patch 1. This form may be round, rectangular or square, but other forms are also possible. The dimensions may also vary greatly, with a length in the range of e.g. 5 cm to 100 cm, and a width in the range of 5 cm to 100 cm, depending on application. The dimensions are not however restricted by this. The sensors may also be made roll-to-roll, i.e. the length is practically unlimited. One example of a typical dimension of a sensor patch 1 for measuring movements of a human knee is for example 5 cm x 10 cm, while for measuring a neck it is 1 cm x 20 cm. A typical sensor patch 1 for measuring movements of a joint of a leg of an adult cow or horse may for example take the form of a sock, collar or sleeve. A typical sensor patch 1 for measuring movements of a lower back of an adult person is for example 10 cm x 100 cm. It is also possible to provide a single (long) patch for measuring the entire spinal column. Such a sensor patch 1 may also have greater dimensions.

The mass of the sensor patch is very low and it is almost flush with the surface. In a specific example, the density of the substrate is 55 g/m² for a thickness of 50 µm, i.e. for a patch of 10 x 100 cm, the substrate weighs (0.1 × 1 × 55 =) 5.5 g. In a specific example, the density of the sensor mat is 1100 kg/m³, i.e. for a strip of 1 × 100 cm this results in a weight of (0.01 × 1 × 40^{∗}10⁻⁶ x 1100 =) 0.44 g.

This force is necessary amongst others to overcome the tension of the elastic film layer 2, the elastic DEAP strips 3 or other capacitative strips or resistive monofilaments, and the elastic protective layer 15, and where applicable also (depending on precise configuration) the tension of the elastic electrical connections and the elastic battery (where fitted).

Thanks to this low force, the user does not notice or scarcely notices the presence of the sensor patch 1, and his freedom of movement is not or is only slightly restricted. Also the risk of detachment of the adhesion layer from the skin (because of the low shear forces) is thus minimised.

Preferably, the thickness of the sensor patch 1 over at least 80%, preferably over its entire surface area, is less than a few millimetres, in some cases even less than 100 µm. The substrate may here for example be thinner than 50 µm, for example only around 30 µm. The thickness of the sensor itself in this example may vary between 40 and 20 µm.

The thickness is determined amongst others depending on application. The thickness may also be influenced by other components which are integrated, such as amongst others possibly the use of so-called "printable electronics". This makes the sensor patch 1 almost flush with the skin on which it is to be applied, and consequently the user perceives no or scarcely any hindrance due to the presence of the sensor patch 1. Also animals do not feel the sensor patch 1 as a "block on their leg", so they are not inclined to want to remove the patch. The patch may also take the form of a sock, collar or sleeve.

In the embodiment of figure 3, the sensor patch comprises merely capacitative strips e.g. three DEAP strips 3, but fewer than three DEAP strips, e.g. only one or only two or more than three DEAP strips, e.g. at least four or at least six or at least eight or at least ten DEAP strips 3 are also possible. If the DEAP strips 3 have a small width Ws of e.g. maximum 5 mm or maximum 4 mm or maximum 3 mm, it is also possible to provide a relatively large number of DEAP strips 3 on the surface of the film layer 2. The strips may be applied e.g. parallel to each other and at a relatively small mutual distance 32 from each other, e.g. of the order of 5 mm to 15 mm. This allows a great concentration of measurement values, whereby a movement of e.g. a joint can be precisely mapped. This aspect is an important advantage of the sensor patch 1 of the present invention, because such a large concentration of measurements is not easily possible with the measuring system from the prior art. If for example one measurement line is provided per 5 mm in both directions X, Y, then 100 movement lines can be measured with a sensor patch measuring 300 mm x 200 mm. One example of a sensor in which a two-dimensional measurement may usefully be used is a back sensor, wherein a herringbone pattern may be used. As specified above, also other capacitative strips or elastic resistive monofilaments may be used.

In a specific example as shown in figure 22, the sensor patch comprises an elastic measurement strip of which the sensor arms are in a cross shape. In such an example, there are four sensor arms which are configured to define a cross shape. This configuration allows the different components of the sensor to be optimally integrated, such as in the present example the self-adhesive substrate (transparent and hence not visible in figure 22), the displacement sensors on the arms, a wireless data transmission system, printed batteries, a system for wireless charging and a microchip which is typically in the centre. In some embodiments, the sensor can comprise several of these measurement strips, wherein the sensor configuration is such that a set of adjacent cross-shaped measurement strips is formed. One example of this is shown in figure 23.

Another important advantage of narrow capacitative strips is that the capacitance value is smaller, whereby less energy is required to charge or discharge the capacitor, and hence measure the capacitance. This means that for a given energy quantity (e.g. the same battery), more measurements can be made if narrower strips are used, whereby e.g. a longer measurement is possible or the measurement frequency can be increased.

Figure 4 shows another embodiment of a sensor patch 1 according to the present invention. Similar electronics to those described above may be used (apart perhaps from a larger package with more pins for the integrated circuit 4, for connecting the larger number of elastic connections to the eight DEAP strips 3). The electronics are not the focus of this figure and are therefore represented by black blocks without showing further details.

Figure 4 shows a sensor patch 1 with two groups each of four parallel DEAP strips 3, wherein the four strips 3 of the first group are mutually parallel and the four strips 3 of the second group are mutually parallel, but wherein the strips of the first group and the strips of the second group stand almost perpendicular to each other. In the embodiment shown, the DEAP strips form a pattern resembling a herringbone. However it is not necessary for the present invention for the number of DEAP strips 3 of the first group to be the same as the number of DEAP strips 3 of the second group, nor is it necessary for the DEAP strips 3 of the first group to stand perpendicular to the DEAP strips 3 of the second group. An orthogonal position of the strips allows for underlying movement to be mapped more precisely, irrespective of the direction in which the skin is stretched. Once again, instead of DEAP strips, other capacitative strips may be used here, or in other embodiments also elastic resistive monofilaments may be used.

The strips or filaments are connected to the electronics (in the black blocks) by means of stretchable and flexible connections 11, as shown e.g. in more detail in figure 6. In the embodiment of figure 4, the first group (e.g. at the top) of four parallel DEAP strips 3 is controlled/read by a first electronics block (at the top), and the second group (at the bottom) of four parallel DEAP strips 3 is controlled/read by a second electronics block (at the bottom), but this is not necessary and preferably all DEAP strips 3 are controlled by one and the same electronics block with only one integrated circuit 4.

Figure 5 shows a diagrammatic drawing of a man with a sensor patch 1 attached to his back. The sensor patch 1 has relatively large dimensions (e.g. 20 cm x 100 cm) but other dimensions are also possible. The DEAP strips, capacitative strips or resistive monofilaments are positioned in a herringbone structure as described for figure 4. The strips or monofilaments 3 shown all have an angle of around to the length direction of the spinal column Y, but the invention is not restricted to this and other angles may also be used. The distance between the strips 3 shown is relatively large (e.g. around 15 cm) but it will be clear that it is also possible to place the strips 3 much closer together, provided there is sufficient space for the wiring. Where applicable, several separate sensor patches 1 may be applied to the same back, wherein each sensor patch covers only a part of the spinal column e.g. half or one third.

Figure 6 shows in more detail an example of the flexible, stretchable and bendable electrical connections of figure 4 between the strips 3 or monofilaments and the integrated circuit 4. Such connections are known in the prior art and may e.g. be formed by printing a zigzag track on the film layer 2. In the specific embodiment of figure 6, the zigzag track consists primarily of an interconnection of horseshoe-shaped elements, but this is not absolutely necessary for the invention and other forms are also possible.

Figure 7 shows an example of a foldable (but not stretchable) substrate with an integrated circuit and some peripheral components (e.g. resistors, capacitors, a clock module and similar) as known in the prior art, as an example of how flexible a substrate with electronic components can be. Such electronics are sometimes also called "flexible electronics" or also "printable electronics". In some embodiments, substrates may also be provided with an integrated circuit which is both bendable and stretchable. Since the dimensions of a human leg, or a leg of a cow or a horse or other livestock, are substantially greater than the dimensions of this integrated circuit, it will be clear to the expert that even if a small part (e.g. less than 20%) of the surface area of the sensor patch 1 where the electronics are situated is not stretchable, this need not affect the stretchability and bendability of the rest of the sensor patch 1, above all if the electronic components are placed at or close to an edge of the sensor patch 1. In the example of figure 4 e.g. zone "A" is stretchable and bendable, and this is the zone where the strips 3 or filaments are situated. Zones "B" are not stretchable, but this is also not necessary if this zone is applied to a part of the skin for which the stretch need not be determined (e.g. next to a joint).

Figure 8 shows an example of a "flexible and stretchable battery" known in the prior art, more specifically from "http://engineering.illinois.edu/ news/article/2013-02-28-stretchable-battery-flexible-circuits", but other so-called "flexible batteries" may also be used. It is an advantage of embodiments in which the energy source comprises several small cells (e.g. capacitors) compared with one large cell, and these cells are connected together by means of flexible connections, because in this way the entire sensor patch 1 retains its flexibility, stretchability and bendability and the thickness can be kept small. In addition it is possible to configure the energy source (e.g. by using parallelism) such that if one elastic connection breaks, energy can still be supplied from the other cells 19.

Figure 9 shows the basic elements of an electrical capacitor as is generally known in electronics. The capacitor shown consists of two conductive plates (conductors) between which is a dielectric. When a voltage V is applied over the plates, a specific charge Q will be distributed over the plates. The capacitance of this structure is defined as the quantity of charge divided by the applied voltage, and is a constant for this structure. The capacitance C may e.g. be determined by applying a known voltage V over the plates (e.g. 3.0 V) and measuring the resulting charge Q (e.g. by then removing the voltage V and replacing it by a resistor, and measuring the current which flows through the resistor). It is noted here that it is not necessary to allow the entire charge to disappear, and that a partial discharge may suffice. Then e.g. a known second voltage (e.g. 1.5 V) can be applied in series with the resistor, and the capacitance value derived from the transient behaviour of the discharge. The capacitor can then be recharged to 3.0 V again and the capacitance determined again. In this way a great deal of energy can be saved, or for the same quantity of energy several capacitance measurements can be performed (e.g. by increasing the measurement frequency), or measurements can be carried out over a longer period before the energy source requires charging (in the case of a rechargeable energy source). A capacitor is a basic component in electronics and need not be described in further detail here.

Figure 10 shows an example of a specific structure with a dielectric electro-active polymer (DEAP) 12 arranged between two thin conductive layers 17a, 17b which function as "electrodes". Because the conductive layers are provided with a ripple structure or a wave structure, the entire structure of figure 10 is stretchable and bendable. Such elastic structures are known in the art and are commercially available from "Danfoss-PolyPower" amongst others, where a silicone dielectric material is sandwiched between two rippled metal film layers (e.g. of silver). The thickness of the elastomer may be of the order of 40 µm. The wave form and the metal film may be applied by coating. The metal film may e.g. be around 100 nm thick. The following link gives more information of one specific embodiment of such material: "http://www.polypower.com/Technology/Overview/", but the invention is not restricted to this one material and other materials or structures with similar properties may also be used, e.g. later generations of this material.

Although the material in itself is not novel, the application of narrow strips thereof which are attached to a flexible film layer 2 to form the sensor patch 1 according to the present invention as described above, is indeed novel. The great advantage is that this allows a high density of measurement lines.

Said structure from Danfoss-PolyPower shows a very stable signal-stretch curve as shown in figure 10 (right). The material retains its properties even after stretching and shrinking more than thousands of times. The stretch can be measured both on straight and on curved surfaces. The stretch may be up to 100%. When the DEAP strip 3 is stretched mechanically, the length increases and the thickness reduces, and the electrical capacitance increases. The stretch of the structure can thus be determined very precisely by measuring the signal, e.g. the capacitance. Measurements with a frequency of up to around 100 Hz are possible. This allows e.g. calculation of the speed of stretch of the DEAP strip 3, which corresponds to the speed of stretching of the skin and of the underlying movement of e.g. the joint.

A preferred embodiment of the sensor patch 1 according to the present invention uses DEAP strips 3 from "Danfoss-PolyPower" as a material for the elastic oblong strips 3.

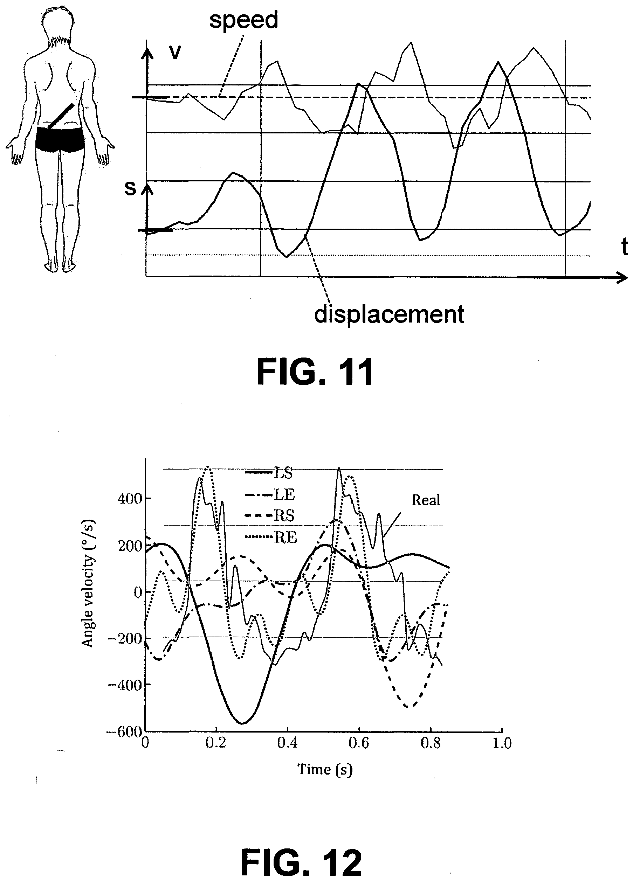

Figure 11 (left) shows a person with a sensor patch 1 applied to the back. This sensor patch 1 may have the form of the sensor patch 1 according to figure 3, but in this specific example has only one single elastic oblong capacitive strip 3 as shown and described in figure 10. The capacitance of this strip is measured at a frequency of approximately 25 Hz, the capacitance is converted into a length measurement using a curve shown in figure 10 (right). The displacement is measured during repeated raising of the knee. The resulting length is shown as a continuous (dark) curve marked "displacement" in figure 11, which shows the stretch of the skin at the back where the sensor patch 1 is applied, and in the direction determined by the DEAP strip 3 (not visible), shown as a function of time. The resulting length measurements (although derived from capacitance values) are regarded as basic data. From these basic data, derived data may be determined such as the movement speed, which can be calculated as the first derivative over time of the displacement curve. Typically, the capacitance of the DEAP strips 3 is measured (sampled) with a frequency from 10 Hz to 100 Hz, e.g. at a frequency of around 25 Hz. Processing of the signal, for example determination of the derivative, may take place both in the sensor itself and in the computer system.

In the use of a sensor patch for monitoring the movement of a back, the sensor patch may also be used for controlling the timing and/or place for administration of spinal electro-stimulation. The patch may for example monitor the movement of a person. If a person suffers pain when making a movement, this will be visible in the movement pattern because the person typically interrupts or changes the movement. This data may be used to control the timing of and precise place where electro-stimulation is best applied. In some embodiments, the present invention therefore concerns a sensor for example as described in an embodiment of the present invention, wherein the sensor allows monitoring of the movement of the back and wherein the sensor comprises a processor coupled directly or wirelessly to the sensor, which allows, for example on the basis of sudden changes in the movement pattern, identification of pain moments and positions on the basis of the movement pattern. Such a system may be coupled to a spinal electro-stimulation system and can send control signals to such a system, so that the spinal electro-stimulation can be applied in a more controlled manner in time and space (position on the back). This leads to a more durable treatment of patients.

In practice, the curve in figure 10 (right) for the DEAP strips 3 from "Danfoss-PolyPower" appears to be slightly temperature-dependent. This temperature dependency may however be greatly reduced or almost completely corrected by using temperature compensation. For this, the integrated circuit 4 preferably comprises a temperature sensor and the necessary software for the processor to take account of the temperature dependency (e.g. by means of a reference table or a mathematical formula). Tests have shown that the temperature dependency can be almost completely eliminated in this manner.

As already stated, the sensor patch may also comprise another sensor such as a temperature sensor. In some embodiments, the sensor patch may therefore comprise a combination of a DEAP sensor and a temperature sensor.

As stated earlier, elastic oblong resistive monofilaments may be used as an alternative to the sensor patch.

According to some embodiments, the present invention also comprises a sensor patch characterised in that it contains a temperature sensor. The temperature sensor is typically attached to an elastic and electrically isolating film layer with a stretchability of at least 100% in all directions in the plane of the film layer X, Y, preferably at least 200% or even at least 300%. A suitable material for this film layer may for example be Tegaderm by 3M or EU50 by Smith & Nephew, although the film layer is not restricted to these materials. The thickness of the film layer is dependent on the application. For a back sensor, a thinner film layer will be used, while for an animal application, a thick patch on a base of sticky gel is used. In a specific embodiment, the present invention thus comprises a sensor patch based on an elastic and electrically isolating film layer, a temperature sensor which is attached to the film layer, and a sticky gel. This allows the application of a sensor via a patch on an animal skin in a stable manner (for example for at least 24 hours). Alternatively, the temperature sensor may also be embedded in an elastic material such as a textile or textile-like material, or an elastomer, in one embodiment for example by means of a sock which is positioned over a limb of the living subject.

Figure 12 shows an example of the angular speed of the forelegs, measured on the basis of displacement sensors, and the actual movement (reality). The subtle variations visible in the actual movement (reality) are identifiable using the sensor patches in accordance with the present invention. It is here an advantage that the rapid variations which occur in reality can be measured with such sensor patches. The sensor patch 1 can thus be used to analyse movement performed at the back or another joint during certain activities (e.g. raising the knee), but the invention is not restricted to this and similar measurements can be performed for any type of activity. Further analysis is also possible as will be described below.

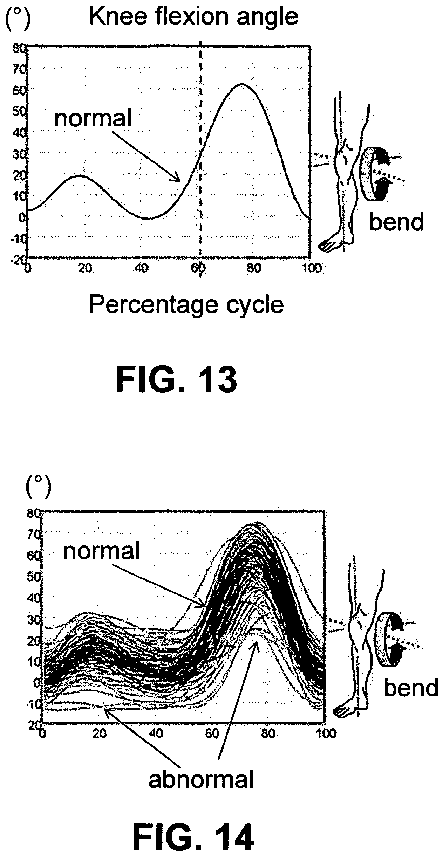

Figure 13 shows an exemplary graph of a normal percentage cycle of measurements of a knee flexion angle (or knee bending angle) during the normal gait (normal walking or stepping) of a person. The curve shown concerns one cycle of a step of a person.

Figure 14 shows several graphs as in figure 13 measured over several moments, for example minutes or days or weeks apart. The different measurements are here shown in the same graph to limit the number of illustrations. Comparison of the graph in figure 14 with that in figure 13, which may serve as a reference graph, allows "abnormalities" to be established irrespective of their cause, e.g. acute problems or slow deterioration. It will be clear that there may be various causes for an abnormal gait or step, such as acute pain as a result of an injury or wound, but also indirect causes such as fatigue or similar.

Figure 15 shows a cow and indicates possible interesting locations at which a sensor patch 1 according to the present invention may be attached for step analysis. To measure panting, which is also a useful parameter for determining animal well-being, for example a sensor can be applied to the chest. Also to measure prenatal contractions, a patch can be applied to the lower abdomen. This allows identification of imminent calving. The sensor may in some embodiments be connected to an analysis and notification system, to inform the appropriate persons of the impending calving.

Figure 16 is a diagrammatic depiction of the forelegs 21 and hind legs 22 of the cow in figure 15. More specifically, the joints of the legs 21, 22 are indicated diagrammatically.

Figure 17 shows different positions on the forelegs of an animal during the walking of a cow for which measurements are performed. Separate graphs were produced for each specific joint of the foreleg or hind leg of the cow during normal gait (normal stepping). This produced several graphs (one per joint of each leg). To measure the graphs, different sensor patches 1 according to the present invention were applied. It will be clear that the sensor patches 1 need not all have the same dimensions and/or the same number of strips 3 or monofilaments, but this may indeed be the case. It is also possible to apply several sensor patches 1 at the same time to the skin of one cow and perform the measurements simultaneously. In this context it is pointed out that it is also perfectly possible to read out the data from all sensor patches virtually simultaneously, for example via Bluetooth, firstly because the necessary bit rate is very low (e.g. of the order of 50 Hz x 16 bits = 800 bits/second per strip or monofilament). Since Bluetooth works in burst mode and with frequency hopping, this data transmission is no problem, but other RF protocols may also be used.

A comparison between the movement of a specific joint of e.g. the left foreleg, and the same joint on the right foreleg, may for example be able to supply information that the animal has suffered a specific injury at the joint concerned. But the analysis can go much further than this. Figure 18 gives a depiction of the vertical displacement versus the horizontal displacement of the development of the stepping pattern of a left front hoof of the cow (only 3 step movements are mapped). Such graphs can be determined by "translating" the position of several joints into the position of the hoof, using a mathematical model and/or a biomechanical model of the legs of the cow. By measuring and storing such characteristics and by comparing the development of different "snapshots" (e.g. one week later or one month later), a clinical picture can be determined since the development of the stepping pattern is specific and indicative of different clinical pictures. In other words, a development can be determined over time, and this development is typically characteristic of a healthy animal or of specific disorders in animals. The sensors according to the present invention, in other words, allow an analysis of the time development of the stepping behaviour. Figure 19 shows why it is important to observe the "stepping pattern" of an animal, more specifically cattle. One of the reasons is early detection of lameness (or injury), but the analysis can go much further than this. Namely there appears to be a correlation between lameness and various other aspects which are important in livestock farming, such as e.g. an influence as a result of infection or as a result of feeding. Hence by measuring the stepping pattern of one or more animals, other important facets can be derived which are related to livestock farming, and these can be corrected in good time.

Figure 20 shows an example of a sensor system 25 according to embodiments of the present invention. This shows an embodiment of a sensor patch 1 as described above, which is attached to a skin of a person or an animal and which performs measurements and stores the measurement data in a local memory. In certain embodiments, the sensor patch 1 has an RF transmitter unit with which the data can be transmitted to a processing system 33. The data can be transmitted virtually instantaneously (e.g. with a delay of less than 10 seconds) or if desired may only take place on request (e.g. only in the morning during milking), depending on implementation. The measurement data (e.g. the "displacements") may then be correlated further to actual biomechanical movements of the joint concerned or the back or other limb of the person or animal concerned on which the measurement was made, by using a mathematical model and a biomechanical database.

in some embodiments, the output from the sensors is stored on the sensor patch and where applicable preprocessed. This preprocessing may comprise the deletion/combination of silent episodes, encryption, the use of data packets for bursting etc. In some cases, an energy management program may also run on a chip in the patch which for example can partly control the transmission. In some embodiments, the data are stored on a chip in the patch and only downloaded at the end of observation. Sometimes transmission may take place in phases, sometimes data may be transmitted directly. The data may be processed on a chip in the patch, but is preferably processed by a fast processor at another location.

The processing system 33 may e.g. be a computer or a laptop with Bluetooth functionality and provided with the necessary software for reading the data from the stretchable sensor patch 1. This computer or laptop differs from known computers and laptops because it contains a specific software program for further processing and interpreting the data, by analysing the measurement data (or derived data such as speed, acceleration etc.) using a mathematical model coupled to a specific biomechanical database relating to the specific joints (or back or similar) of the subject (e.g. a person or a cow or horse). The result of this analysis can be shown on the display or monitor or screen, and/or stored in a file, and/or printed out, and/or transmitted via a network.

Instead of a computer or laptop however, other e.g. smaller portable devices may be used for reading the sensor patch 1, e.g. a Smartphone or a PDA or a tablet or similar, provided with Bluetooth functionality and the necessary software. The data can then be transmitted further from the laptop or portable device to other interested parties, e.g. medical personnel.

Embodiments of the sensor patch 1 according to the present invention may suitably be used for measuring prenatal contractions, for example for an animal such as cattle, e.g. a cow or a horse. The sensor patch 1 is preferably applied to the skin.

Embodiments of the sensor patch 1 according to the present invention may also suitably be used for measuring step movements (the gait) of an animal or person. Here one or more sensor patches 1 are attached to the skin of an animal, namely on at least one of the joints of at least one of the legs 21, 22 of the animal.

Embodiments of the sensor patch 1 according to the present invention may also suitably be used for measuring the step movements (the gait) of an animal in order to detect the first signs of lameness or injury, an indication that may have a damaging influence on the entire European cattle stock. Early treatment can drastically improve the cost efficiency of animal husbandry.

Embodiments of the sensor patch 1 according to the present invention may also suitably be used for measuring the movements of the back of a person or animal, i.e. for measuring movements of the spinal column. One or more sensor patches 1 are here attached to the skin at the level of the spinal column, e.g. at the lower back or at the neck, or at both places simultaneously.

The sensor patch 1 is preferably attached to the skin in pre-stretched form. To apply the sensor patch 1 with a suitable pretension (not too much and not too little), optionally an elliptical figure (drawing or printing) may be applied to the stretchable protective layer 15 of the sensor patch 1, which forms a circle in the ideal pre-stretched state. In this way the sensor patch 1 can easily be applied with the suitable pretension. However the pretension is not critical and the sensor patch will also work with slightly more or slightly less or even no pretension. The advantage of applying the sensor patch 1 with pretension is that it is also possible to measure negative stretch (i.e. shrinkage of the skin).

In this way, for example the information flow obtained with a sensor patch according to the present invention may take place as follows. Using the sensors, signals are measured. These are stored locally and transmitted at suitable times. This transmission may take place for example locally (low-energy transmission) such as to a local mobile device, such as a mobile phone or Smartphone. For example, a user interface for obtaining preliminary information may already be provided on this mobile device. From the mobile device, the information may be sent over a network to a data processing unit where the information can be processed further. The processed information can then be distributed further to the direct users, to medical staff, domestic help etc. The specific interface at which the processed information can be offered, and the information offered, may be application-specific.

Also, different phases of processing and presentation of information may take place at different times.

Whereas, in the description above, reference is made to a sensor patch in the conventional form of a patch, the sensor patch also relate to a sock, collar or sleeve with an integrated sensor patch. In one specific example, a sensor patch is provided in the form of a sock, which for example can be used for monitoring and/or analysing the movement of a leg of an animal. In specific applications, for example the movement of a leg of a horse can be monitored, for example during training or analysis of the walking behaviour of the horse. In the exemplary sensor patch, a polymer stretch sensor is provided which is mounted between an upper and a lower textile strip. Controllers, processors for processing measurement results, electrical contacts, and batteries may be contained in the textile strips. Alternatively, these may be mounted thereon. The sensor may be attached to the textile strips in any manner such as for example via hook and loop connections, such as Velcro connections. Electrical connectors may also be provided. Furthermore, sensor patches according to the present invention may also contain an identification element such as an RFID element, a barcode etc. which can be identified remotely, or another identification element as known to the person skilled in the art. This not only allows identification of the sensor but also coupling of the cycles performed to the life of the sensor.

In specific embodiments , the sensor system thus concerns a sock, collar or sleeve to be placed over a limb of a person or animal, wherein the sock, collar or sleeve is typically made of an elastic material such as for example a textile or a textile-like material or an elastomer material, wherein the sensor is embedded or attached. Embedding or attaching as stated above may take place in any manner whatsoever. The sock, collar or sleeve may be provided with an adhesive edge on the top and/or underside of the sock, collar or sleeve, which can counter the slipping of the sock, collar or sleeve after this has been placed over the limb. The limb may be an arm or a leg of the living subject such as a person, an animal, such as more specifically a horse.

The sock, collar or sleeve may typically be provided for placing over an elbow or knee joint. It is an advantage of the present embodiment that a good connection of the sock, collar or sleeve over the limb is achieved, such that measurement can be carried out which accurately records the movement of the limb.

The sensor which is embedded in the elastic material or attached to the elastic material comprises at least one elastic oblong capacitive strip, wherein the capacitative strip comprises a dielectric electro-active polymer. This capacitative strip may have a stretchability of at least 50% in the length direction. Further properties of the capacitative strip correspond to those in the embodiments or aspects described above.

When embedding or attaching the sensor to the elastic sock, collar or sleeve, it is not necessary for an elastic and electrically isolating film layer to be present, such as is present for embodiments of the conventional form of a patch. In some examples the sensor may be intertwined with the elastic material, e.g. the textile or textile-like material, or it may for example be connected to the sock, collar or sleeve at its end or at the ends and at some intermediate points.

In embodiments according to the present invention, one or more processors and/or controllers may be embedded in the sock, collar or sleeve, also electrical connectors for connecting the sensor to one or more processors, and energy source (rechargeable or otherwise), a signal transmission system for transmitting data and/or signals to a remote processing and/or execution system etc. These components may have similar characteristics to those described above in other embodiments of the present invention.

In some embodiments based on a sock, collar or sleeve, a secondary sensor may be included, for example at a controller, although embodiments of the present invention are not limited by this. Such a secondary sensor in some embodiments is a MEMS sensor (micro-electromechanical sensor). The sensor may be configured to carry out one or more measurements of acceleration, inclination, gravity, temperature, air pressure and capacitance. Such a sensor may be used for example to achieve an automatic calibration of the DEAP sensor, also to perform auto-corrections to compensate for any shift or other effects which may occur on measurement with the DEAP sensor.

In some embodiments, results of measurements with the DEAP sensor are correlated with results measured with another sensor, such as for example results measured with a MEMS sensor. Such correlated data typically provides a better picture of the measured action, movement etc. than when only one sensor is used. Here the accurate measurement of rapid movements which is obtained by means of the DEAP sensor is combined with other measurements.

Embodiments as described above may be used in a multiplicity of applications including monitoring of athletes, treatment and monitoring of animals such as racehorses etc.

The present invention also concerns the use of a sock, collar or sleeve-based sensor as described above for monitoring living subjects. This use is particularly advantageous for monitoring movements which are carried out at very high speed, such as for example the throwing of a ball, the execution of a golf swing etc. Information obtained in this way may for example include angles between the top part and the bottom part of a limb, but also accelerations, speeds and relative positions of the body, such as for example a limb. One specific application is monitoring of competition animals such as for example horses. This monitoring may serve for example for mapping the movement before and/or after treatment, or for depicting the movement of an animal during a competition. Such monitoring may for example take place in track racing or endurance competitions, although use is not restricted to this.

In one aspect, a garment with an integrated sensor, wherein the integrated sensor comprises at least one elastic oblong capacitive strip, wherein the capacitative strip comprises a dielectric electro-active polymer. This capacitative strip may have a stretchability of at least 50% in the length direction. Further properties of the capacitative strip may correspond to those in embodiments or aspects described above. When embedding or attaching a sensor to the garment, for example the elastic e.g. textile or textile-like garment, there is therefore no need for an elastic and electrically isolating film layer to be present, such as is present for embodiments in the conventional form of a patch. The sensor may in some examples be interwoven with the elastic material, such as for example the textile or textile-like material or an elastomer material, or may for example be connected (stitched) to the sock, collar or sleeve at its ends or at the ends and some intermediate points. The garment may be any garment which covers a part for which movement must be measured or monitored. It may for example be a T-shirt or trousers. In some embodiments of the present invention, the garment is situated close to the body, whereby the movement of the body can be followed more accurately. In some embodiments, the garment may be a sock, collar or sleeve. As in the embodiments described above, components may be attached or embedded for wireless data transmission, electrical connection, energy supply, signal processing etc., and also additional sensors. Similar embodiments and advantages can thus be obtained. The sensor system may comprise, as well as a measurement strip, other sensors such as an inertia measurement system which may be used for calibration purposes, as a crosscheck or to obtain secondary data.

It is an advantage of the sensors according to embodiments of the present invention that as well as highly dynamic data information, information can also be captured concerning physiology which is representative for example for the reaction of a muscle.

Embodiments of the present invention may be provided in an absolute body reference framework, so that no account need be taken of drifting, a very accurate location determination is possible with good reproducibility (changes as small as 100 µm can be perceived) and a very high sample rate (1000 Hz or higher).

Embodiments i.e. wherein sensors are incorporated in a garment or wherein sensor patches are used according to embodiments described in this application, may for example be used for measuring a specific attitude during an activity (such as curvature of a back), for measuring the movement of a specific person, for identifying respiration, for example for measuring a respiration pattern etc. This may be applied for example for sports clothing but also in consumer products wherein, via an application, information concerning back health can be supplied to the user. It may also be used for monitoring rapid movements or for dynamic capturing of 3D movements, for example of a backbone.

In embodiments in aspects of the present invention, different methods may be used for calibrating the sensor, e.g. DEAP sensor, and where applicable correcting this during use. Some of the calibration techniques are described below.

In a first example, a second sensor is also present in the system, such as for example a micro-electromechanical sensor or another sensor which can supply movement information. This movement information may for example be used to calibrate the sensor by automatic calibration, for example on start-up of the system. Movement information obtained simultaneously with the second sensor and the (first) sensor is then correlated, from which calibration information is obtained for the (first) sensor. This utilises the fact that second sensors can supply very accurate measurements for some movement patterns (for example if the movement is not too slow so that it is measurable, or also not too fast so the movement can be followed). Measurements in this regime can then be used to calibrate the sensor. This then allows a calibrated sensor to be used, also for movement modes where the (first) sensor supplies good results while this is not the case for the second sensor. Typical modes in which the (first) sensor scores significantly well/better include very slow and/or fast movements. The same sensor also applies good result for modes in which the second sensors also supply good results.

In a second example, the sensor may be calibrated by an optical system. Such a calibration may take place for example by carrying out simultaneous measurements with the sensor and an optical system. Such an optical system can for example monitor a number of points, intrinsic points or attached calibration points. The monitored data may then, for example after data processing, supply movement information which can be used to calibrate the sensor. Alternatively, the optical system can record video images which, where applicable after video analysis and data processing, supply suitable movement information. In one example wherein the movement of a limb is followed, for example a cylinder can be fitted to the top part of the limb and a second cylinder fitted to the bottom part of the limb, from which for example an angle can then be derived. This information can in turn be used to calibrate the sensor.

The obtained calibration information may be 2D information, for example the relative angle between the different parts of the limb in one plane, but also 3D information.

In yet another aspect, an elastic object for the performance of physical exercises for body training or rehabilitation. The elastic object may for example be a ball, although other forms are also possible. The elastic object is suitable for being compressed as part of physical exercises. The elastic object comprises a sensor which contains at least one elastic oblong capacitive strip, wherein the capacitative strip comprises a dielectric electro-active polymer and/or at least one elastic oblong resistive filament e.g. a monofilament. The sensor may be attached as a patch to the elastic object. Alternatively the sensor may be integrated in the elastic object, for example in the elastic material from which the object is made. The sensor can thus be stuck on, be interwoven, fused in or otherwise connected inseparably. Further properties may be those as described for example for the sensor patch.

By way of illustration and without embodiments being limited thereby, one specific example is an elastic object and a training system which includes such an elastic object, as shown in figure 21. The elastic object 2100 in the present example is an air-filled rubber ball on which a sensor is present as described in embodiments of the aspects described above. The sensor can accurately map the rapid kinetics of movement such as hitting the top of the ball, squeezing the ball with two arms or two legs etc. The sensor data in the present example may be processed in a real-time data processor, which for example may be integrated in a specific chip. The elastic object 2100 is typically connected, for example wirelessly connected, to a processor and a user interface 2110 which for example may be a screen. In some embodiments, such a user interface may be a graphical user interface 2110. In the example given, such a graphical user interface 2110 displays graphical information 2112 concerning which hand should be used (left hand or right hand), graphical information 2114 concerning the duration of the exercise (time elapsed, time for the exercise or part thereof etc.), graphical information 2116 concerning the force which should be applied. The graphical user interface 2110 may however also contain other information such as the number of exercises still remaining, an indication of the exercise just performed, an indication of the number of calories burned etc. There may therefore be a two-way communication between the sensor and the processor. The system may furthermore also comprise a supporting surface 2120 for the elastic object, the height of the supporting surface being adjustable as a function of the user. Identification means for identifying the user may be provided.

The processor or controller of the system may be configured to offer a particular set of exercises on the basis of identification of the user. After training, a comparison with earlier results may be made, and for example the effect of training, long-term use of medication etc. can be seen.

It is an advantage of the present embodiments that pressure as a function of time can be measured extremely accurately, both in amplitude and resolution.

Another aspect concerns the same concept of an elastic object with a sensor positioned thereon or integrated therein. This may for example be intended to determine pressure or pressure distribution in the elastic object or another object sitting around the elastic object. One specific example of use concerns the evaluation of arthroplasty, an orthopaedic surgical procedure in which the surface of a musculoskeletal joint is replaced, modified or aligned. By using the sensor patch, small anomalies can be detected such as asymmetric stepping or incorrect distribution of weight over the joints. Another aspect in which the embodiments of the present invention may be used is the detection of reduced coordination. This for example allows prevention of falls. Another example of use of a sensor according to embodiments of the present invention is the early detection of Alzheimer's disease.

In a particular aspect, the present invention concerns a system for assistance in knee arthroplasty. In one aspect, the system concerns a kit with a balancer for arthroplasty of a knee, and a thin load sensor which sits in such a balancer. One example of a balancer is shown in figure 24.

The thin load sensor is placed as a disc below the springs of the balancer and the displacement then measured in the thin load sensor is related to the pressure. The data from the load sensor are also processed as a left-right compression of the springs of the balancer (and also the angle of the top section relative to the bottom).

The kit of elements may also comprise a set of controllers which can be applied to the lower leg and upper leg, for example MEMS-IMU systems. This allows good positioning of the leg. A combination of the information from the load sensor and from these controllers allows the knee angle to be derived with great precision from the spatial information. This information can then be used for optimum adjustment of the balancer.

In order to combine the balancer with the load sensor, the balancer is typically configured so that the load sensor can be placed below the springs of the balancer. In addition the balancer may also comprise a clip which can ensure that the measuring element is not loaded until intervention is applied, since this would be harmful to the measurement element. The clip may for example be a clip placed between the springs and the load sensor before intervention begins, which can be removed at the start of intervention.

The thin load sensor may for example be a measurement strip on a substrate which for example may be elastic and electrically isolating. The measurement strip may comprise an oblong capacitive strip with a dielectric electro-active polymer, and have a stretchability of at least 50% in the length direction. Alternatively or in combination therewith, the measurement strip may comprise an elastic oblong resistive filament with a thermoplastic elastomer with a resistivity which changes as a function of the length of the filament. Further aspects of the sensor and measurement strip may be as described in other embodiments.

The specific form of the sensor may typically be adapted to fit the balancer.

The end result is that when the balancer is fitted in the knee, the left-right compression and the angle are measured fully automatically (as a function of the angle) and everything can be logged precisely. The specific angle between the plates as a function of the angle of the knee as can be measured is illustrated in figure 25.

The present invention also concerns the use of a load sensor as described above for recording an angle between elements of a balancer during knee arthroplasty as a function of the knee angle.

### Reference numerals

- 1: Sensor patch
- 2: Film layer
- 3: DEAP strip
- 4: Integrated circuit
- 6: Energy source
- 7: Adhesion layer
- 8: Person
- 11: Stretchable electrical connections
- 12: DEAP material
- 15: Protective layer
- 17: Metal film layer
- 19: Energy cell (e.g. capacitor)
- 20: Cow
- 21: Foreleg
- 22: Hind leg
- 25: Sensor system
- 26: Receiver
- 27: Calculator unit
- 28: Mathematical model
- 29: Biomechanical database
- 30: Display, screen
- 32: Distance between parallel DEAP strips
- 33: Processing system
- Ls: Length of the strip
- Ws: Width of the strip
- L: Length of the film layer/patch
- W: Width of the film layer/patch

## Claims

1. An elastic sensor patch (1) comprising
- an elastic and electrically isolating film layer (2) with a stretchability of at least 100% in all directions in a plane of the film layer (X, Y), and
- a plurality of elastic measurement strips attached to the elastic film layer (2), wherein each elastic measurement strip comprises an oblong capacitive strip (3) with a dielectric electro-active polymer which has a stretchability of at least 50% in its length direction,
**characterized in that** the sensor patch comprises a first number of at least two elastic measurement strips (3), which are oriented mutually parallel to each other in a first direction and lie at a distance from each other in the range of 3 mm to 100 mm, and a second number of at least two elastic measurement strips (3), which are oriented mutually parallel to each other in a second direction which intersects the first direction, wherein each elastic measurement strip comprises two electrically conductive metal film layers (17) attached to opposite sides of the oblong capacitive strip with the dielectric electro-active polymer, wherein the two metal film layers (17) have a geometric form which allows elastic elongation without breaking the metal film.

2. An elastic sensor patch according to claim 1 wherein the elastic measurement strips are positioned in a herringbone structure.

3. The sensor patch (1) according to any of the preceding claims, wherein the width (Ws) of the measurement strip is less than 5 mm.

4. The sensor patch (1) according to any of the preceding claims, wherein the sensor patch furthermore comprises an adhesion layer (7) for sticking the film layer (1) to a skin of a person or animal.

5. The sensor patch (1) according to any of the preceding claims, wherein the sensor patch has a mass (m) of less than 100 g, preferably less than 50 g, and/or wherein the force necessary to stretch the sensor patch by 50% in any direction in the plane of the sensor patch (X, Y) is less than 2.0 Newtons, and/or wherein the sensor patch has a thickness (d) which over its entire surface is less than 10 mm, for example less than 1 mm.

6. A sensor system (25) comprising
- the sensor patch (1) according to any of the preceding claims 1 to 5;
- a processing system (33) with a receiver (26) for receiving data transmitted by the sensor patch (1), and with a calculator unit (27) for processing the received data, and with a read unit (30) for displaying the processed data.

7. A sensor system (25) according to claim 6, wherein the system furthermore comprises a MEMs inertia measurement unit.

8. A sensor system (25) according to one of claims 6 or 7, wherein the processing system is configured to receive simultaneous measurements from the sensor patch and a calibration system and to combine these to calibrate the sensor patch.

9. A sensor system (25) according to claim 8, wherein the calibration system is an opto-technological mapping system.

10. Use of the sensor patch (1) according to any of claims 1 to 5 or of a sensor system according to any of claims 6 to 9, wherein the sensor patch (1) is applied to the skin of a person (8), namely on the back at the level of the backbone, for measuring movements of the back.

11. The use according to claim 10, wherein the sensor patch (1) is applied to said skin in a pre-stretched form.

## Patentansprüche

1. Ein elastisches Sensorpflaster (1), das umfasst
- °eine elastische und elektrisch isolierende Folienschicht (2) mit einer Streckbarkeit von mindestens 100 % in alle Richtungen in einer Ebene der Folienschicht (X, Y), und
- °eine Vielzahl elastischer Messstreifen, die an der elastischen Folienschicht (2) angebracht ist, wobei jeder elastische Messstreifen einen länglichen kapazitiven Streifen (3) mit einem dielektrischen elektroaktiven Polymer umfasst, der eine Streckbarkeit von mindestens 50 % in seiner Längsrichtung aufweist,
**dadurch gekennzeichnet, dass** das Sensorpflaster eine erste Anzahl aus mindestens zwei elastischen Messstreifen (3) umfasst, die zueinander parallel in einer ersten Richtung ausgerichtet sind und in einem Abstand voneinander in dem Bereich von 3 mm bis 100 mm liegen, und eine zweite Anzahl aus mindestens zwei elastischen Messstreifen (3), die zueinander parallel in einer zweiten Richtung ausgerichtet sind, die die erste Richtung schneidet, wobei jeder elastische Messstreifen zwei elektrisch leitende Metallfolienschichten (17) umfasst, die an entgegengesetzten Seiten des länglichen kapazitiven Streifens mit dem dielektrischen elektroaktiven Polymer angebracht sind, wobei die zwei Metallfolienschichten (17) eine geometrische Form aufweisen, die elastische Dehnung ohne Brechen der Metallfolie erlaubt.

2. Ein elastisches Sensorpflaster nach Anspruch 1, wobei die elastischen Messstreifen in einer Fischgrätmusterstruktur positioniert sind.

3. Ein Sensorpflaster (1) nach einem der vorstehenden Ansprüche, wobei die Breite (Ws) des Messstreifens kleiner als 5 mm ist.

4. Ein Sensorpflaster (1) nach einem der vorstehenden Ansprüche, wobei das Sensorpflaster weiter eine Haftschicht (7) zum Ankleben der Folienschicht (1) an einer Haut einer Person oder eines Tiers umfasst.

5. Ein Sensorpflaster (1) nach einem der vorstehenden Ansprüche, wobei das Sensorpflaster eine Masse (m) von weniger als 100 g, bevorzugt weniger als 50 g aufweist, und/oder wobei die Kraft, die zum Strecken des Sensorpflasters um 50 % in eine Richtung in der Ebene des Sensorpflasters (X, Y) erforderlich ist, kleiner als 2,0 Newton ist, und/oder wobei das Sensorpflaster eine Dicke (d) aufweist, die über seine gesamte Oberfläche kleiner als 10 mm, zum Beispiel kleiner als 1 mm ist.

6. Ein Sensorsystem (25) das umfasst
- °das Sensorpflaster (1) nach einem der vorstehenden Ansprüche 1 bis 5,
- °ein Verarbeitungssystem (33) mit einem Empfänger (26) zum Empfangen von Daten, die von dem Sensorpflaster (1) übertragen werden, und mit einer Rechnereinheit (27) zum Verarbeiten der empfangenen Daten, und mit einer Leseeinheit (30) zum Anzeigen der verarbeiteten Daten.

7. Ein Sensorsystem (25) nach Anspruch 6, wobei das System weiter eine MEMs-Trägheitsmesseinheit umfasst.

8. Ein Sensorsystem (25) nach einem der Ansprüche 6 oder 7, wobei das Verarbeitungssystem dazu konfiguriert ist, gleichzeitige Messungen von dem Sensorpflaster und einem Kalibrierungssystem zu empfangen, und diese zu kombinieren, um das Sensorpflaster zu kalibrieren.

9. Ein Sensorsystem (25) nach Anspruch 8, wobei das Kalibrierungssystem ein optotechnologisches Abbildungssystem ist.

10. Verwendung des Sensorpflasters (1) nach einem der Ansprüche 1 bis 5 oder eines Sensorsystems nach einem der Ansprüche 6 bis 9, wobei das Sensorpflaster (1) an die Haut einer Person (8), insbesondere auf dem Rücken auf dem Niveau des Rückgrats zum Messen von Bewegungen des Rückens angelegt wird.

11. Die Verwendung nach Anspruch 10, wobei das Sensorpflaster (1) an die Haut in einer vorgestreckten Form angelegt wird.

## Revendications

1. Une pastille de détection élastique (1) comprenant
- une couche de film élastique et d'isolation électrique (2) avec une extensibilité d'au moins 100 % dans toutes les directions dans un plan de la couche de film (X, Y), et
- une pluralité de bandelettes de mesure élastiques fixées à la couche de film élastique (2), dans laquelle chaque bandelette de mesure élastique comprend une bandelette capacitive oblongue (3) avec un polymère électro-actif diélectrique qui présente une extensibilité d'au moins 50 % dans le sens de sa longueur,
**caractérisée en ce que** la pastille de détection comprend un premier nombre d'au moins deux bandelettes de mesure élastiques (3), qui sont orientées mutuellement parallèles l'une à l'autre dans une première direction et reposent à une certaine distance l'une de l'autre dans la plage de 3 mm à 100 mm, et un second nombre d'au moins deux bandelettes de mesure élastiques (3), qui sont orientées mutuellement parallèles l'une à l'autre dans une seconde direction qui croise la première direction, dans laquelle chaque bandelette de mesure élastique comprend deux couches de film métallique électriquement conductrices (17) fixées à des côtés opposés de la bandelette capacitive oblongue avec le polymère électro-actif diélectrique, dans laquelle les deux couches de film métallique (17) présentent une forme géométrique qui permet un allongement élastique sans rupture du film métallique.

2. Une pastille de détection élastique selon la revendication 1, dans laquelle les bandelettes de mesure élastiques sont positionnées dans une structure en chevron.

3. Une pastille de détection (1) selon l'une quelconque des revendications précédentes, dans laquelle la largeur (Ws) de la bandelette de mesure est inférieure à 5 mm.

4. Une pastille de détection (1) selon l'une quelconque des revendications précédentes, dans laquelle la pastille de détection comprend en outre une couche d'adhérence (7) pour coller la couche de film (1) sur une peau d'une personne ou d'un animal.

5. Une pastille de détection (1) selon l'une quelconque des revendications précédentes, dans laquelle la pastille de détection présente une masse (m) inférieure à 100 g, de préférence inférieure à 50 g, et/ou dans laquelle la force nécessaire pour étirer la pastille de détection de 50 % dans une quelconque direction dans le plan de la pastille de détection (X, Y) est inférieure à 2,0 Newtons, et/ou dans laquelle la pastille de détection présente une épaisseur (d) qui sur l'ensemble de sa surface est inférieure à 10 mm, par exemple inférieure à 1 mm.

6. Un système de détection (25) comprenant
- la pastille de détection (1) selon l'une quelconque des revendications 1 à 5 précédentes ;
- un système de traitement (33) avec un récepteur (26) destiné à recevoir des données transmises par la pastille de détection (1), et avec une unité de calcul (27) destinée à traiter les données reçues, et avec une unité de lecture (30) destinée à afficher les données traitées.

7. Un système de détection (25) selon la revendication 6, dans lequel le système comprend par ailleurs une unité de mesure d'inertie MEMS.

8. Un système de détection (25) selon l'une des revendications 6 ou 7, dans lequel le système de traitement est configuré pour recevoir simultanément des mesures en provenance de la pastille de détection et d'un système d'étalonnage et pour les combiner pour étalonner la pastille de détection.

9. Un système de détection (25) selon la revendication 8, dans lequel le système d'étalonnage est un système de mappage opto-technologique.

10. Utilisation de la pastille de détection (1) selon l'une quelconque des revendications 1 à 5 ou d'un système de détection selon l'une quelconque des revendications 6 à 9, dans laquelle la pastille de détection (1) est appliqué sur la peau d'une personne (8), à savoir sur le dos au niveau de la colonne vertébrale, pour mesurer des mouvements du dos.

11. L'utilisation selon la revendication 10, dans laquelle la pastille de détection (1) est appliquée sur ladite peau sous forme préétirée.
